# EUROPÄISCHE PATENTSCHRIFT

(11) **EP 2 335 675 B1**
(45) Veröffentlichungstag und Bekanntmachung des Hinweises auf die Patenterteilung: **18.02.2015**
(21) Anmeldenummer: 09015330.5
(22) Anmeldetag: 10.12.2009
(51) Int. Cl.: A61K 8/04, A61K 8/06, A61K 8/34, A61K 8/36, A61K 8/37, A61K 8/81, A61Q 19/00

(54) **Emulgatorfreie, Polymer-stabilisierte Schaumformulierungen**
Emulsifier-free, polymer stabilised foam formulas
Formules de mousse sans émulsifiant et stabilisées par polymères

(43) Veröffentlichungstag der Anmeldung: 22.06.2011
(73) Patentinhaber: Neubourg Skin Care GmbH & Co. KG, 48268 Greven (DE)
(72) Erfinder: Daniels, Rolf, Prof. Dr., 72108 Rottenburg a. N. (DE)
(74) Vertreter: Maiwald, Walter

(56) Entgegenhaltungen:
- EP-A1- 0 835 647
- WO-A2-2006/065530
- WO-A2-2008/138894
- DE-A1- 19 825 961
- DE-A1- 19 907 715
- DE-A1- 19 923 648
- US-A- 3 330 730
- US-A1- 2003 211 061
- LOEFFLER ET AL: "A new ph stable polymer for gels and o/w emulsions", SOFW-JOURNAL SEIFEN, OELE, FETTE, WACHSE, VERLAG FUR CHEMISCHE INDUSTRIE, AUGSBURG, DE, Bd. 128, 1. Januar 2002 (2002-01-01), Seiten 46-52, XP009128608, ISSN: 0942-7694
- ANONYM: "Taking care of your sensory innovations, Aristoflex AVC and Aristoflex HMB", INTERNET CITATION, 2003, XP002372730, Gefunden im Internet: URL:http://www.clariant-surfactants.com/fu n/e2wtools.nsf/vwLookupDownload s/Taking_care_of_your_Sensory_Innovations_ -_Aristoflex_-_2003:pdf/$FI LE/Taking_care_of_your_Sensory_Innovations _-_Aristoflex_-_2003.pdf [gefunden am 2006-03-16]
- ANONYMOUS: 'Duden | sinnfällig | Rechtschreibung, Bedeutung, Definition, Synonyme', [Online] 30 Oktober 2013, XP055086030 Gefunden im Internet: <URL:http://www.duden.de/rechtschreibung/si nnfaellig> [gefunden am 2013-10-30]

## Beschreibung

### Technisches Gebiet der Erfindung

Die vorliegende Erfindung betrifft kosmetische und dermatologische Schaumformulierungen, insbesondere Schaumcremes, auf Basis von Emulsionen vom Typ Öl-in-Wasser, die frei sind oder im Wesentlichen frei sind von herkömmlichen Emulgatoren, und die mindestens ein grenzflächenaktives, ionisches Polymer mit einem Molekulargewicht von größer als 5000 g/mol umfassen, wobei das ionische Polymer ein Copolymer ist, das als Monomereinheiten ein ionisches Monomer und mindestens ein weiteres Monomer enthält.

### Hintergrund der Erfindung

### 1. Emulsionen

Unter Emulsion versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (liphophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Der Begriff "Emulgator" bzw. "herkömmlicher Emulgator" ist im Stand der Technik bekannt. Herkömmliche Emulgatoren und ihre Verwendung werden beispielsweise in den Publikationen: Pflegekosmetik, 4. Auflage, Wissenschaftliche Verlagsgesellschaft mbH Stuttgart, Seiten 151 bis 159, und Fiedler Lexikon der Hilfsstoffe, 5. Auflage Editio Cantor Verlag Aulendorf, Seiten 97 bis 121, beschrieben.

Herkömmliche Emulgatoren können entsprechend ihrem hydrophilen Molekülteil in ionische (anionische, kationische und amphotere) Emulgatoren und nicht-ionische Emulgatoren untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nicht-ionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristika aller im System enthaltenen Stoffe zu berücksichtigen. Betrachtet man z. B. Hautpflegeemulsionen, so können polare Ölkomponenten wie z. B. UV-Filter zu Instabilitäten führen. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar, welcher in den unterschiedlichsten Anwendungsgebieten eingesetzt wird. So stehen für die Pflege der Haut, insbesondere für die Rückfettung trockener Haut, eine Vielzahl von Produkten - wie Lotionen und Cremes - zur Verfügung. Ziel der Hautpflege ist es, den durch tägliches Waschen verursachten Fett- und Wasserverlust der Haut auszugleichen. Außerdem sollen Hautpflegeprodukte vor Umwelteinflüssen - insbesondere vor Sonne und Wind - schützen und die Hautalterung verzögern.

Kosmetische Emulsionen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Auch zur Reinigung der Haut und Hautanhangsgebilde finden Emulsionen in Form von Reinigungsemulsionen Verwendung. Sie werden meist zur Gesichtsreinigung und insbesondere zur Entfernung von dekorativer Kosmetik eingesetzt. Derartige Reinigungsemulsionen haben - im Gegensatz zu anderen Reinigungszubereitungen wie beispielsweise Seife - den Vorteil, besonders hautverträglich zu sein, da sie in ihrer lipophilen Phase pflegende Öle und/oder unpolare Wirkstoffe - wie z.B. Vitamin E - enthalten können.

### 2. Emulgatorfreie Emulsionen

Die IUPAC definiert den Begriff "Emulgator" wie folgt: Emulgatoren sind grenzflächenaktive Substanzen. Sie halten sich bevorzugt in der Grenzfläche zwischen Öl- und Wasserphase auf und senken dadurch die Grenzflächenspannung. Emulgatoren erleichtern bereits bei niedriger Konzentration die Emulsionsbildung. Darüber hinaus vermögen diese Substanzen die Stabilität von Emulsionen zu verbessern, indem sie die Geschwindigkeit der Aggregation und/oder der Koaleszenz verringern.

Zur Stabilisierung pharmazeutischer und kosmetischer Emulsionen werden überwiegend sogenannte echte Emulgatoren, d.h. herkömmliche Emulgatoren im Sinne der vorliegenden Beschreibung, eingesetzt, die ihrer Struktur und ihrem physikalisch-chemischen Verhalten nach zur Stoffklasse der Tenside zählen. Sie zeichnen sich durch einen amphiphilen Aufbau und die Fähigkeit zur Mizellenassoziation aus.

Solche niedermolekularen, amphiphilen Substanzen werden jedoch immer wieder als Ursache für Unverträglichkeiten bei Hautpflegeprodukten genannt, wie z.B. eine Störung der Hautbarriere oder Mallorcaakne. Daher sucht die kosmetische Industrie nach Alternativen zu den konventionellen Formulierungen in Form von emulgatorfreien Emulsionen.

Emulgatorfreie Emulsionen sind frei von Emulgatoren im herkömmlichen Sinne, d.h. von amphiphilen Substanzen mit niedriger Molmasse (d.h. Molmasse < 5000 g/mol), die in geeigneten Konzentrationen Mizellen und/oder andere flüssigkristalline Aggregate ausbilden können.

Der Begriff "emulgatorfrei" ist im Fachgebiet etabliert. Nach einer im interdisziplinären Konsens zwischen Pharmazeuten, Dermatologen und anderen Fachleuten verabschiedeten Begriffsdefinition der Gesellschaft für Dermopharmazie (http://www.dermotopics.de/german/ausgabe_1_03_d/emulgatorfrei_1_2003_d.htm) kann eine Formulierung als "emulgatorfrei" bezeichnet werden, wenn sie statt mit Emulgatoren im engeren Sinne (d.h. herkömmlichen Emulgatoren) mit grenzflächenaktiven Makromolekülen (Molmasse von über 5000 g/mol) stabilisiert ist.

Als vielversprechender Ansatz für emulgatorfreie Emulsionen, mit dem Ziel, ausreichend stabile und kosmetisch ansprechende Produkte zu erhalten, die die mit herkömmlichen Emulgatoren verbundenen Nachteile vermeiden helfen, hat sich der Einsatz von Polymer- sowie von Feststoffemulgatoren erwiesen.

### 3. Feststoff-stabilisierte Emulsionen

Ein Beispiel für emulgatorfreie Emulsionen sind Feststoff-stabilisierte Emulsionen. Feststoff-stabilisierte Emulsionen, die im Fachgebiet auch als Pickering-Emulsionen bezeichnet werden, sind durch feinstverteilte Feststoffteilchen stabilisiert und ermöglichen es, auf herkömmliche Emulgatoren weitestgehend zu verzichten.

In Feststoff-stabilisierten Emulsionen kommt es zu einer Anreicherung des festen Stoffes an der Phasengrenze Öl/Wasser in Form einer Schicht, wodurch ein Zusammenfließen der dispersen Phase verhindert wird.

Geeignete Feststoffemulgatoren sind insbesondere partikuläre, anorganische oder organische Feststoffe, die sowohl von hydrophilen als auch von lipophilen Flüssigkeiten benetzbar sind. Bevorzugt werden in den Feststoff-stabilisierten Emulsionen oder Pickering-Emulsionen z.B. Titandioxid, Zinkoxid, Siliziumdioxid, Fe₂O₃, Veegum, Bentonit oder Ethylcellulose als Feststoffemulgatoren eingesetzt.

### 4. Polymer-stabilisierte Emulsionen

Ein weiteres Beispiel emulgatorfreier Emulsionen sind Polymer-stabilisierte Emulsionen. Bei Polymer-stabilisierten Emulsionen erfolgt die notwendige Stabilisierung im Gegensatz zu den klassischen Emulsionen nicht mit amphiphilen, tensidartigen Emulgatoren, sondern mit Hilfe geeigneter Makromoleküle. Derartig stabilisierte Formulierungen unterscheiden sich in ihrem Irritationspotenzial deutlich von den mit herkömmlichen Emulgatoren stabilisierten Emulsionen. Aufgrund ihrer hohen Molmasse können polymere Emulgatoren nicht in das Stratum corneum penetrieren. Unerwünschte Wechselwirkungen, z.B. im Sinne einer Mallorca-Akne, sind deshalb nicht zu erwarten.

Werden Polymere zugesetzt, so resultiert ihr stabilisierender Effekt häufig durch ihren Verdickungseffekt und dadurch, dass sie der Außenphase der Emulsion eine Fließgrenze verleihen.

Wesentlich effektiver ist der Einsatz grenzflächenaktiver Makromoleküle, wie z.B. Carbomer 1342 oder Hydroxypropylmethylcellulose als primäre Emulgatoren. Diese bilden strukturierte Grenzflächenfilme, die einen effektiven Koaleszenzschutz gewährleisten. Die Erhöhung der Viskosität der Außenphase spielt in diesem Fall für die Stabilität der Emulsionen eine untergeordnete Rolle.

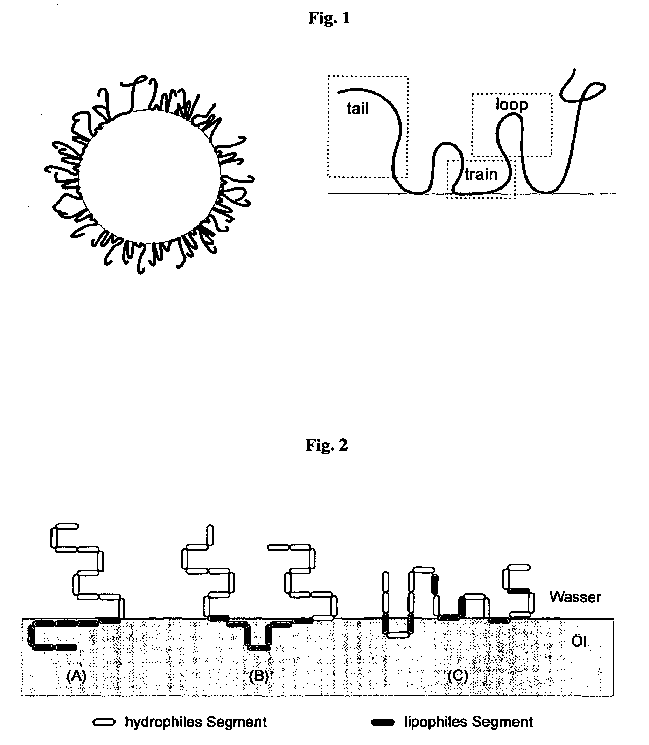

Der Aufbau des von Polymeremulgatoren gebildeten Grenzflächenfilms lässt sich ganz allgemein mit dem sogenannten Tail-Loop-Train-Modell beschreiben (siehe Myers D., Polymers at Interfaces, in Meyers D.: Surfaces, interfaces and colloids. VHC Publishers New York, Seiten 283-297, 1991), das in Figur 1 schematisch abgebildet ist.

Polymere können dann als Emulgatoren eingesetzt werden, wenn sie eine genügend hohe Grenzflächenaktivität aufweisen. Besonders geeignet sind Copolymere mit hohem Molekulargewicht, die einen Monomerenanteil geringerer Polarität zusätzlich zu einem hydrophilen Monomerenanteil enthalten. Dadurch bewirken sie neben einer Viskositätserhöhung in der kontinuierlichen Wasserphase gleichzeitig und hauptsächlich eine Stabilisierung der Öl-/Wasser-Phasengrenzfläche. Der Anteil geringerer Polarität adsorbiert an die Ölphase, und das hydrophile Gerüst quillt in der Wasserphase unter Ausbildung einer Gelstruktur an der Phasengrenzfläche. Die aus den stark hydratisierten, hydrophilen Polymersegmenten entstandene Gelstruktur, z. B. in Form kleiner Geltröpfchen entlang der Öl-/Wasser-Phasengrenzfläche, kann gegebenenfalls einen noch effektiveren Koaleszenzschutz ermöglichen als ein nicht gel-artiger Grenzflächenfilm, wie ihn beispielsweise Hydroxypropylmethylcellulose ausbildet.

Die genaue molekulare Anordnung der Copolymer-Emulgatoren an der Phasengrenzfläche wird maßgeblich von der Verteilung hydrophiler und weniger polarer Segmente über das gesamte Copolymer-Molekül bestimmt. In Figur 2 sind die hierbei möglichen Anordnungen für A-B-Blockcopolymere [Fig. 2 (A)], A-B-A-Blockcopolymere [Fig. 2, (B)], und Copolymere mit statistisch verteilten hydrophilen Segmenten und Segmenten geringerer Polarität [Fig. 2, (C)] schematisch wiedergegeben.

Ohne an eine Theorie gebunden zu sein, wird davon ausgegangen, dass insbesondere quervernetzte ionische Copolymere eine besonders gute Stabilisierung der Emulsionen erzielen. Dies lässt sich darauf zurückführen, dass die Quervernetzung eine starke Entfaltung der hydrophilen (ionischen) Polymersegmente in der Wasserphase verhindert. Das hydrophile (ionische) Polymersegment kann sich also nicht beliebig in die Wasserphase ausdehnen, sondern behält eine kompakte Struktur und muss nahe der Öl-/Wasser-Phasengrenzfläche hydratisieren und quellen. Dadurch entsteht eine besonders rigide, tröpfchenförmige Gelstruktur an der Öl-/Wasser-Phasengrenzfläche, die einen optimalen Koaleszenzschutz und Stabilisierungseffekt bewirkt.

Eine Stabilisierung von Öl in Wasser Emulsionen unter Ausbildung der beschriebenen Gelstruktur an der Öl-/Wasser-Phasengrenzfläche bewirkt beispielsweise der Polymeremulgator Carbomer 1342. Carbomer 1342 ist ein Copolymer aus Acrylsäure und C₁₀-C₃₀-Alkylacrylaten, wobei der hydrophile Acrylsäure-Anteil den lipophilen Alkylacrylat-Anteil überwiegt. Die C₁₀-C₃₀-Alkylacrylate sind zusätzlich mit Allylpentaerithrol quervernetzt.

Besonders effektive Stabilisatoren für emulgatorfreie Öl in Wasser Emulsionen sind verschiedene, kommerziell erhältliche Copolymere der 2-Acrylamido-2-methyl-propansulfonsäure.

### 2-Acrylamido-2-methyl-propansulfonsäure mit der chemischen Formel (2)

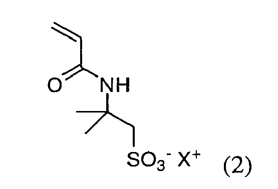

wobei X⁺ im Falle der freien Säure H⁺ ist, wird auch als AMPS oder 2-Methyl-2-[1-oxo-2-propenyl)amino]-1-propansulfonsäure bezeichnet. Ihre Salze (bei denen X⁺ein anderes Kation als H⁺ repräsentiert) werden auch als Acryloyldimethyltaurate bezeichnet.

Zu dieser Familie besonders geeigneter Copolymere zählen die kommerziell erhältlichen Polymere Aristoflex® AVC und Aristoflex® HMB der Firma Clariant. Aristoflex® AVC (INCI-Name Ammonium Acryloyldimethyltaurat/Vinylpyrrolidon Copolymer) und Aristoflex® HMB (INCI-Name Ammonium Acryloyldimethyltaurat / Beheneth-25 Methacrylat Crosspolymer) enthalten einen ionischen Monomeranteil, 2-Acrylamido-2-methyl-propansulfonsäure (AMPS), sowie eine weiteren, weniger polaren Monomeranteil (Vinylpyrrolidon bzw. Beheneth-25 Methacrylat). Diese Polymere werden als Verdickungsmittel und Stabilisator für Öl in Wasser Emulsionen verwendet, die bereits in geringer Konzentration extrem stabile Emulsionen bilden. Insbesondere können diese Polymere mit nahezu jeder Ölphase verwendet werden, umfassend Silikonöle, Kohlenwasserstoffe/Wachse und Esteröle. Außerdem können sie über einen breiten pH-Bereich angewendet werden (Aristoflex® AVC: pH 4,0 bis 9,0; Aristoflex® HMB: pH 3,0 bis 9,0), und sind UV-stabil.

Copolymere der 2-Acrylamido-2-methyl-propansulfonsäure werden auch von der Firma Seppic als Polymer-Emulgatoren für emulgatorfreie Emulsionen angeboten bzw. entwickelt. Dazu zählen das kommerziell erhältliche Produkt Sepinov^{™} EMT 10 (INCI Name Hydroxyethylacrylat /Natriumacryloyldimethyltaurat Copolymer, CAS-Nummer 111286-86-3), sowie die experimentellen Polymere 8732MP (Produktname: Sepinov P88, ein Natriumacrylat /Acryloyldimethyltaurat /Dimethylacrylamid Crosspolymer, CAS-Nummer 187725-30-0), 8885MP2 (Produktname: Sepinov EG-P, ein Natriumacrylat/ Natriumacryloyldimethyltaurat Copolymer, CAS-Nummer 77019-71-7), und 8947MP. Diese Polymere besitzen ebenfalls gute emulgierende Eigenschaften, sind über einen breiten pH-Bereich einsetzbar (z.B. für Sepinov^{™} EMT 10 pH 3-11), und UV-stabil.

Aristoflex® AVC, Aristoflex® HMB, Sepinov^{™} EMT 10 und die experimentellen Seppic-Polymere 8732MP, 8885MP2 und 8947 MP werden in bereits neutralisierter Form formuliert, d.h. die 2-Acrylamido-2-methyl-propansulfonsäure-Einheit liegt in der Formulierung zumindest partiell als Salz vor, und liegen pulverförmig vor.

Acryloyldimethyltaurat-Copolymere, deren Herstellung, sowie deren Verwendung als Verdicker bzw. Stabilisator von Emulsionen für kosmetische und pharmazeutische Anwendungen, werden beispielsweise in den Veröffentlichungen EP 1 069 142 A1, EP 1 116 733 A1, WO 2008/087326 A2 und EP 1 138 703 A1 beschrieben.

WO 03/022236 A1 beschreibt die Verwendung von Taurat-Copolymeren, insbesondere von Aristoflex® AVC, als Verdickungsmittel und Stabilisator für kosmetische Zusammensetzungen, insbesondere Lotionen und Creme-Formulierungen, enthaltend C₁-C₂₅ alpha- oder beta-Hydroxycarbonsäuren.

### 5. Schaumformulierungen

Eine besondere Anwendungsform kosmetischer und/oder dermatologischer Emulsionen ist die Applikation als Schäume. Schaumformulierungen haben den Vorteil, sich leicht auf der Haut verteilen zu lassen. Die schaumige Konsistenz wird als angenehm empfunden und die Produkte hinterlassen in der Regel ein gutes Hautgefühl. Insbesondere wirkt sich aber auch die physikalische Struktur des Schaumes positiv auf die Hautschutzfunktion aus. Schäume sind komplizierte physikalische Gebilde, die einer besonderen Abstimmung der den Schaum bildenden Komponenten bedürfen. Schäume werden generell durch Versprühen einer Emulsionsformulierung oder einer wässrigen Tensid-(Stabilisator-)lösung erhalten. Beispielsweise wird die mit Treibgas versetzte Emulsion aus einem unter Druck stehenden Behältnis abgegeben (solche Systeme werden in der Literatur und Patentliteratur auch als Aerosolschäume bezeichnet). Dabei expandiert das unter Druck stehende Gemisch aus Emulsion und Treibgas und bildet die Schaumbläschen. Insbesondere kommt es zu einer Expansion der dispersen Ölphase, in welcher das öllösliche Gas gelöst ist. Schäume können aber auch mit Hilfe anderer Systeme erzeugt werden, wie beispielsweise Pumpsprays.

Abgestimmte Schaumformulierungen weisen eine stabile zwei- oder mehrphasige polydisperse Struktur bei der Applikation auf, die auf der Haut eine Netzstruktur, vergleichbar mit einer Membran bildet. Solche Netzstrukturen haben den Vorteil, dass diese eine Schutzfunktion, beispielsweise vor Kontakt mit Wasser ausbilden, jedoch einen ungehinderten Gasaustausch mit der Umgebung zulassen. Bei solchen Schäumen kommt es praktisch zu keiner Behinderung der *Perspiratio insensibiles* und keinem entsprechenden Wärmestau. Damit werden die positiven Eigenschaften einer Schutz- und Pflegefunktion mit einer unveränderten Hautatmung verbunden.

Bisher bekannte Schaumformulierungen enthalten meist herkömmliche Tenside/Emulgatoren, die für die Stabilisierung der Emulsion und für die darauf folgende Schaumstabilität sorgen.

Herkömmliche Emulgatoren bzw. Tenside werden jedoch, wie bereits zuvor diskutiert, immer wieder als Ursache für Unverträglichkeiten bei Hautpflegeprodukten genannt. Auf den Zusatz geeigneter Stabilisatoren kann allerdings nicht gänzlich verzichtet werden, da disperse Systeme, wie bereits beschrieben, beispielsweise Emulsionen thermodynamisch instabil sind.

Die oben genannten Pickering-Emulsionen sind eine Möglichkeit, herkömmliche Emulgatoren zu vermeiden. In der EP 1 352 639 A1 bzw. der DE 101 62 840 werden Pickering-Emulsionen vorgestellt, die jedoch als Emulsionen in Form von Lotionen, Cremes und Gelen zum Einsatz kommen.

In der WO 2004/017930 sind weitere Pickering-Emulsionen beschrieben, die sich insbesondere durch eine niedrige Viskosität auszeichnen und damit für dermatologische Tücher geeignet sind. Solche dünnflüssigen Pickering-Emulsionen lassen sich sogar unter einer Nebelbildung versprühen.

In der WO 2008/138894 werden Schaumformulierungen auf Basis von emulgatorfreien Pickering-Emulsionen beschrieben.

Die WO 2008/155389 beschreibt Schaumformulierungen auf Basis von Emulsionen, deren Ölphase mindestens eine membranbildende Substanz umfasst, die in der Schaumformulierung lamellar angeordnete Membranen ausbildet, wobei die Emulsionen bevorzugt emulgatorfrei sind.

Keines der oben beschriebenen Dokumente beschreibt jedoch Schäumformulierungen auf Basis von emulgatorfreien Emulsionen, die durch ionische, grenzflächenaktive Polymere mit einem Molekulargewicht von größer als 5000 g/mol stabilisiert werden, wobei das ionische Polymer ein Copolymer ist, das als Monomereinheiten ein ionisches Monomer (M1) und mindestens ein weiteres Monomer enthält.

### Zusammenfassung der Erfindung

Die Anmelderin hat nun erkannt, dass sich Öl in Wasser Emulsionen umfassend mindestens einen Feststoffemulgator und mindestens ein grenzflächenaktives, ionisches Polymer mit einem Molekulargewicht von größer als 5000 g/mol, wobei das ionische Polymer ein Copolymer ist, das als Monomereinheiten ein ionisches Monomer (M1) und mindestens ein weiteres Monomer enthält, als Basis für Schaumformulierungen eignen. Dabei werden die positiven Eigenschaften der Schaumformulierungen mit denen Polymer-stabilisierter Emulsionen verbunden. Es lassen sich insbesondere Schaumformulierungen ohne herkömmliche Emulgatoren oder mit sehr geringen Anteilen an herkömmlichen Emulgatoren herstellen, die die positiven Eigenschaften des Schaumes, nämlich die physikalische Struktur und angenehme Anwendbarkeit, mit denen der Polymer-stabilisierten Emulsionen, wie z. B. deren gute Hautverträglichkeit, verbinden. Dies macht solche Schaumformulierung insbesondere verwendbar für kosmetische und dermatologische Formulierungen für empfindliche Hauttypen. Es wird dabei Verträglichkeit und Anwendungskomfort vorteilhaft miteinander verbunden.

Dabei ist es nicht selbstverständlich, dass das Verschäumen von Öl in Wasser Emulsionen umfassend mindestens ein grenzflächenaktives, ionisches Polymer mit einem Molekulargewicht von größer als 5000 g/mol, wobei das ionische Polymer ein Copolymer ist, das als Monomereinheiten ein ionisches Monomer (M1), und mindestens ein weiteres Monomer enthält, zu stabilen Schaumprodukten führt. Schäume werden, wie bereits erwähnt, beispielsweise durch Einarbeiten von (druck)verflüssigten Treibgasen in O/W-Emulsionssysteme erhalten. Verdampft bei dem Verschäumen das in der dispersen Ölphase gelöste Treibgas, bildet sich ein Schaum (Gas-in-Flüssig-Dispersion). Beim Verdampfen bzw. Expandieren des in der dispersen Ölphase gelösten Treibgases kommt es zu einer Dilatation der dispersen Ölphase. Es ist nun überraschend gefunden worden, dass die an der Phasengrenzfläche ausgebildete Polymer-Gelstruktur dieser Dehnungsbelastung stand hält und es beim Verschäumen der erfindungsgemäßen Schaumformulierungen nicht zum Brechen der Zubereitung kommt, und ein zur Anwendung in pharmazeutischen und kosmetischen Produkten geeigneter Schaum ausgebildet wird. Dieser ist stabil genug, um z.B. auf die Haut aufgetragen zu werden.

Inbesondere wurde überraschend gefunden, dass eine Öl in Wasser Emulsion umfassend mindestens ein grenzflächenaktives, ionisches Polymer mit einem Molekulargewicht von größer als 5000 g/mol, wobei das ionische Polymer ein Copolymer ist, das als Monomereinheiten ein ionisches Monomer (M1), und mindestens ein weiteres Monomer enthält, sowie mindestens einen Feststoffemulgator, eine besonders geeignete Basis für Schaumformulierungen darstellt. Die daraus hergestellten Schaumformulierungen weisen insbesondere eine verbesserte Stabilität im Vergleich zu den im Stand der Technik bekannten, aus Pickering-Emulsionen erzeugten Schaumformulierungen, sowie zu den oben erwähnten, ausschließlich Polymer-stabilisierten Schaumformulierungen auf.

Die Erfindung betrifft somit Schäume und Schaumformulierungen wie in den Ansprüchen 1 und 2 definiert.

Ferner betrifft die Erfindung die Verwendung einer im Wesentlichen emulgatorfreien Emulsion gemäß Anspruch 12 für die Herstellung einer Schaumformulierung gemäß einem der Ansprüche 2 bis 11.

Ferner betrifft die Erfindung die Verwendung mindestens eines grenzflächenaktiven, ionischen Polymers gemäß Anspruch 13 zur Stabilisierung von Schaumformulierungen gemäß einem der Ansprüche 2 bis 11.

Ferner betrifft die Erfindung die Verwendung der erfindungsgemäßen Schaumformulierungen gemäß einem der Ansprüche 2 bis 11 als Träger für Wirkstoffe, als Hautpflegemittel, als Hautreinigungsmittel oder als Sonnenschutzmittel. Die Schaumformulierung kann daher zur Herstellung eines Kosmetikums, Medizinprodukts oder Arzneimittels eingesetzt werden.

Außerdem umfasst die Erfindung ein Verfahren zur Herstellung der erfindungsgemäßen Schaumformulierungen gemäß einem der Ansprüche 2 bis 11. Das Verfahren umfasst die Schritte:
a) Herstellen einer Emulsion vom Typ Öl in Wasser
b) Abfüllen der Emulsion mit Treibgas in einen Druckbehälter, oder
c) Abfüllen der Emulsion in einen anderen als einen Druckbehälter, der bei Abgabe der Emulsion einen Schaum erzeugt.

### Beschreibung der Abbildungen

Figur 1 zeigt den schematischen Aufbau eines makromolekularen Grenzflächenfilms nach dem Tail-Loop-Train-Modell.
Figur 2 gibt die mögliche Anordnung eines Copolymer-Emulgators in Abhängigkeit von der Verteilung der lipophilen und hydrophilen Segmente für A-B-Blockcopolymere (A), A-B-A-Blockcopolymere (B) und Copolymere mit statistisch verteilten hydrophilen Segmenten und Segmenten geringerer Polarität (C) schematisch wieder.

### Detaillierte Beschreibung der Erfindung

### 1. Definitionen

Gemäß der vorliegenden Erfindung sind Schaumformulierungen Formulierungen, insbesondere Emulsionen, die zur Erzeugung von Schaum sinnfällig hergerichtet sind. Die Formulierungen können insbesondere entweder mit (druck)verflüssigtem Treibgas in einen Druckbehälter abgefüllt sein oder ohne Treibgas in einen anderen Behälter als einen Druckbehälter abgefüllt sein, der es ermöglicht, bei Abgabe der Formulierung/Emulsion einen Schaum zu erzeugen. Beispielsweise können Pumpspraybehälter verwendet werden.

Gemäß der vorliegenden Erfindung sind im Wesentlichen emulgatorfreie Emulsionen solche Emulsionen, die weniger als 0,5 Gew.-%, vorzugsweise weniger als 0,3 Gew.-%, mehr bevorzugt weniger als 0,1 Gew.-%, besonders bevorzugt weniger als 0,05 Gew.-% herkömmlicher Emulgatoren enthalten. Gemäß der Erfindung sind emulgatorfreie Emulsionen solche, die keine herkömmlichen Emulgatoren enthalten.

Gemäß einem Aspekt sind herkömmliche Emulgatoren gemäß der vorliegenden Erfindung anionische, kationische, amphotere und nichtionische Tenside. Typische Vertreter der anionischen Tenside sind neutralisierte verzweigte und/oder unverzweigte, gesättigte oder ungesättigte Fettsäuren mit einer Kettenlänge von 10 bis 40 Kohlenstoffatomen. Typische Vertreter der kationischen Tenside sind Ammoniumverbindungen. Typische Vertreter der nichtionischen Tenside haben einen hydrophilen Molekülteil, wie etwa Glycerin, Polyglycerin, Sorbitan, Kohlenhydrate bzw. Polyoxyethlenglykole, der über Ester- und/oder Etherbindungen mit dem lipophilen Molekülteil verknüpft ist, der üblicherweise aus Fettalkoholen, Fettsäuren oder Isofettsäuren besteht. Zum Beispiel zählen die polyethoxylierte Fettsäureester mit Kettenlängen von 10 bis 40 Kohlenstoffatomen und mit einem Ethoxylierungsgrad von 5 bis 100 zu dieser Gruppe. Ferner zählen gesättigte und/oder ungesättigte, verzweigte und/oder unverzweigte Fettalkohole mit einer Kettenlänge von 10 bis 40 Kohlenstoffatomen zu den nichtionischen Emulgatoren. Häufig werden herkömmliche Emulgatoren in Kombinationen eingesetzt.

Herkömmliche Emulgatoren im Sinne der vorliegenden Beschreibung sind in den Publikationen: Pflegekosmetik, 4. Auflage, Wissenschaftliche Verlagsgesellschaft mbH Stuttgart, Seiten 151 bis 159 und Fiedler Lexikon der Hilfsstoffe, 5. Auflage, Editio Cantor Verlag Aulendorf, Seiten 97 bis 121, beschrieben.

Gemäß der Erfindung sind herkömmliche Emulgatoren alle amphiphilen Substanzen mit einer Molmasse < 5000 g/mol, die in höherer Konzentration Mizellen bilden können und/oder in der Emulsion nicht als Feststoff vorliegen.

Herkömmliche Emulgatoren liegen bei üblichen Lagerungs- und Anwendungstemperaturen, wie z.B. Raumtemperatur, in der Emulsion nicht als Feststoff vor. Dies bedeutet, dass z.B. die oben beschriebenen Fettalkohole mit einer Kettenlänge von 10 bis 40 Kohlenstoffatomen, soweit sie in einer Emulsion aufgrund deren Formulierung/Zusammensetzung nicht als Feststoff, sondern beispielsweise flüssigkristallin oder gelöst vorliegen, unter die Definition eines herkömmlichen Emulgators fallen. Liegen die Fettalkohole mit einer Kettenlänge von 10 bis 40 Kohlenstoffatomen in der Emulsion hingegen als Feststoff vor, so fallen sie nicht unter die Definition eines herkömmlichen Emulgators.

Gemäß der Erfindung ist ein Feststoffemulgator eine partikuläre Substanz, die sowohl von lipophilen als auch von hydrophilen Flüssigkeiten benetzbar ist. Es kann sich hierbei um anorganische oder organische Feststoffe handeln. Die Partikel können ferner unbehandelt oder beschichtet sein. Bevorzugt liegt die Partikelgröße zwischen 1 nm und 200 nm, mehr bevorzugt zwischen 5 nm und 100 nm. Werden organische Feststoffemulgatoren verwendet, wie beispielsweise kristalline Fettsäuren, kristalline Fettsäureester oder kristalline Fettalkohole, so liegt die Partikelgröße bevorzugt zwischen 1 nm und 1000 nm.

Unter dem Begriff einer freien Säure bzw. einer freien sauren funktionellen Gruppe sind gemäß der Erfindung Verbindungen mit einer Säurefunktion bzw. eine Säurefunktion zu verstehen, die zu mindestens 98%, bevorzugt mindestens 99% , besonders bevorzugt zu 100% nicht neutralisiert vorliegen.

Unter dem Begriff einer vollständig neutralisierten Säure bzw. einer vollständig neutralisierten sauren funktionellen Gruppe sind gemäß der Erfindung Verbindungen mit einer Säurefunktion bzw. eine Säurefunktion zu verstehen, die zu mindestens 98%, bevorzugt mindestens 99% , besonders bevorzugt zu 100% neutralisiert in Form ihrer Salze vorliegen.

Unter dem Begriff einer partiell neutralisierten Säure bzw. einer partiell neutralisierten sauren funktionellen Gruppe sind gemäß der Erfindung Verbindungen mit einer Säurefunktion bzw. eine Säurefunktion zu verstehen, die zu mindestens 2%, bevorzugt mindestens 1%, und zu höchstens 98%, bevorzugt höchstens 99% neutralisiert in Form ihrer Salze vorliegen, während der nicht neutralisierte Teil als freie Säure vorliegt.

"Stabilisierung einer Schaumformulierung" bedeutet gemäß der Erfindung, dass durch die Anwesenheit des Emulgator-Polymers, insbesondere einer Kombination des Emulgator-Polymers mit einem Feststoffemulgator, die Struktur des aus der Schaumformulierung gebildeten Schaumes länger aufrechterhalten werden kann, d.h. für einen Zeitraum von mindestens 1 Minute, besonders bevorzugt mindestens 2 Minuten, bevor der Schaum zerfällt.

### 2. Zusammensetzung der erfindungsgemäßen Schaumformulierungen

Die erfindungsgemäßen Schaumformulierungen basieren auf einer im Wesentlichen emulgatorfreien Emulsion vom Typ Öl in Wasser, umfassend eine Ölphase und eine Wasserphase, wobei die Emulsion mindestens ein grenzflächenaktives, ionisches Polymer mit einem Molekulargewicht von größer als 5000 g/mol umfasst.

Das mindestens eine grenzflächenaktive, ionische Polymer mit einem Molekulargewicht von größer als 5000 g/mol ist ein Copolymer, das als Monomereinheiten ein ionisches Monomer (M1), und mindestens ein weiteres Monomer enthält. Im Folgenden werden derartige Polymere auch als Emulgator-Copolymere bezeichnet.

Zudem umfasst die Öl in Wasser Emulsion ferner mindestens einen Feststoffemulgator.

In einer bevorzugten Ausführungsform enthält die Emulsion keine herkömmlichen Emulgatoren.

Der Ausdruck "Emulgatorsystem bestehend im Wesentlichen aus" bedeutet, dass das Emulgatorsystem gegebenenfalls geringe Mengen herkömmlicher Emulgatoren enthalten darf. Jedoch muss die Menge herkömmlicher Emulgatoren dabei so niedrig sein, dass die Emulsion umfassend das Emulgatorsystem eine "im Wesentlichen emulgatorfreie Emulsion" gemäß der vorliegenden Erfindung ist. In einer bevorzugten Ausführungsform besteht das Emulgatorsystem aus dem mindestens einen Feststoffemulgator und dem mindestens einen Emulgator-(Co)polymer. Neben den Emulgatoren des Emulgatorsystems umfasst die der Schaumformulierung zugrundeliegende Öl in Wasser Emulsion keine weiteren Emulgatoren.

Alternativ oder ergänzend lässt sich das mindestens eine grenzflächenaktive, ionische Polymer mit einem Molekulargewicht von größer als 5000 g/mol beschreiben als ein Polymer, das die Emulsion durch Ausbildung einer Gelstruktur an der Öl-/Wasser-Phasengrenzfläche stabilisiert. Derartige Emulgator-Polymere können anionisch, kationisch oder zwitterionisch sein.

Die an der Öl-/Wasser-Phasengrenzfläche ausgebildete Gelstruktur liegt dabei bevorzugt in Form einer die Ölphase umgebenden Schicht aus Geltröpfchen vor, die vorzugsweise stark hydratisiert sind. Das Vorliegen derartiger Gelstrukturen kann über grenzflächenrheologische Messungen nachgewiesen werden.

In einer weiteren Ausführungsform wirkt das grenzflächenaktive, ionische Polymer, das die Emulsion durch Ausbildung einer Gelstruktur an der Öl-/Wasser-Phasengrenzfläche stabilisiert, zusätzlich als Verdickungsmittel, d.h. neben der Ausbildung einer Gelstruktur an der Öl-/Wasser-Phasengrenzfläche wird außerdem die Viskosität der umgebenden Wasserphase erhöht. Die Gelstruktur an der Öl-/Wasser-Phasengrenzfläche weist dabei bevorzugt eine höhere Viskosität auf als die die Gelstruktur umgebende Wasserphase.

Bei dem mindestens einen grenzflächenaktiven, ionischen Polymer mit einem Molekulargewicht von größer als 5000 g/mol, das die Emulsion durch Ausbildung einer Gelstruktur an der Öl-/Wasser-Phasengrenzfläche stabilisiert, handelt es sich um ein Copolymer, das als Monomereinheiten ein ionisches Monomer (M1), und mindestens ein weiteres Monomer enthält (d.h. um ein Emulgator-Copolymer).

Das mindestens eine grenzflächenaktive, ionische Polymer mit einem Molekulargewicht von größer als 5000 g/mol ist bevorzugt wasserlöslich oder wasserquellbar, besonders bevorzugt wasserquellbar. "Wasserquellbar" bedeutet im Rahmen der vorliegenden Erfindung, dass das Polymer bei Kontakt mit Wasser unter Volumenzunahme hydratisiert.

Bevorzugt ist das mindestens eine grenzflächenaktive, ionische Polymer mit einem Molekulargewicht von größer als 5000 g/mol in der Emulsion in einer Menge von ungefähr 0,01 bis ungefähr 10 Gew.%, bevorzugt von ungefähr 0,05 bis ungefähr 8 Gew.%, mehr bevorzugt von ungefähr 0,1 bis ungefähr 5 Gew.%, ganz besonders bevorzugt von ungefähr 0,2 bis ungefähr 1 Gew.%. bezogen auf das Gesamtgewicht der Emulsion (ohne Treibgas) enthalten.

### Emulgator-Copolymere:

Wie oben diskutiert, enthalten die erfindungsgemäß eingesetzten Emulgator-Copolymere als Monomereinheiten ein ionisches Monomer (M1) und mindestens ein weiteres Monomer. Das mindestens eine weitere Monomer unterscheidet sich dabei von dem ionischen Monomer (M1).

Enthält das Emulgator-Copolymer ein weiteres Monomer, so liegt ein Bipolymer vor, bei zwei weiteren Monomeren ein Terpolymer, usw.. Im Sinne der vorliegenden Erfindung sind Bipolymere, Terpolymere, Quaterpolymere, usw. alle von dem Begriff Copolymer umfasst.

Copolymere, die intramolekulare Quervernetzungen enthalten, werden im Rahmen der vorliegenden Erfindung als quervernetzte Copolymere oder Crosspolymere bezeichnet.

Bevorzugt besitzt das mindestens eine weitere Monomer eine andere Polarität als das ionische Monomer (M1). Der Begriff "Polarität" soll dabei wie im Fachgebiet üblich verstanden werden. Dabei bezeichnet Polarität eine durch Ladungsverschiebung in Atomgruppen entstandene Bindung von getrennten Ladungsschwerpunkten, die bewirken, dass eine Atomgruppe nicht mehr elektrisch neutral ist. Das elektrische Dipolmoment ist ein Maß für die Polarität eines Moleküls. Je nach Größe des Gesamtdipolmoments eines Moleküls, das sich durch vektorielle Addition der einzelnen Dipolmomente ergibt, ist ein Stoff mehr oder weniger polar, wobei der Übergang fließend von extrem polar bis komplett unpolar verläuft. Das weitere Monomer weist beispielsweise eine andere Polarität als das ionische Monomer (M1) auf, wenn es ein nicht-ionisches Monomer ist, das definitionsgemäß eine geringere Polarität besitzt als eine ionische Verbindung. Das weitere Monomer mit anderer Polarität kann aber ebenfalls ein ionisches Monomer sein. Enthält dieses neben seiner ionischen Funktionalität beispielsweise eine lange, hydrophobe Fettsäurekette, so weist es insgesamt eine geringere Polarität auf als ein ionische Monomer (M1), das keinen hydrophoben Bestandteil enthält.

Das mindestens eine weitere Monomer wird vorzugsweise ausgewählt aus der Gruppe bestehend aus ionischen Monomeren, nicht-ionischen Monomeren, und Mischungen davon. Besonders bevorzugt umfasst das mindestens eine weitere Monomer mindestens ein nicht-ionisches Monomer.

### Ionisches Monomer (M1):

Das ionische Monomer (M1) ist vorzugsweise anionisch, kationisch oder zwitterionisch, besonders bevorzugt anionisch.

Vorzugsweise enthält das ionische Monomer (M1) freie, partiell neutralisierte oder vollständig neutralisierte saure funktionelle Gruppen. Ein Monomer enthaltend freie saure funktionelle Gruppen wird deswegen als ionisches Monomer verstanden, weil die sauren funktionellen Gruppen entweder bei der Herstellung des Copolymers oder bei der Herstellung der Öl in Wasser Emulsion zumindest partiell neutralisiert werden können.

Die sauren funktionellen Gruppen sind vorzugsweise ausgewählt aus der Gruppe bestehend aus Sulfonsäuregruppen, Carbonsäuregruppen, Phosphorsäuregruppen, Phosphonsäuregruppen und Mischungen davon.

In einer bevorzugten Ausführungsform wird das ionische Monomer (M1) ausgewählt aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze, Ammoniumsalze oder Alkanolammoniumsalze; oder als Anhydrid vorliegen können, und Mischungen davon.

Dabei ist ist das ionische Monomer (M1) bevorzugt eine Acrylamidoalkylsulfonsäure, wie beispielsweise 2-Acrylamido-2-methyl-propansulfonsäure. Besonders bevorzugt liegt die Acrylamidoalkylsulfonsäure partiell oder vollständig neutralisiert als Alkalimetall-, Erdalkalimetall-, Ammonium- oder Alkanolammoniumsalz vor, besonders bevorzugt als Natrium- oder Ammoniumsalz, ganz besonders bevorzugt als Ammoniumsalz.

Besonders bevorzugt besitzt die Acrylamidoalkylsulfonsäure die allgemeinen Formel (1), 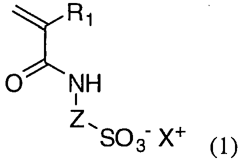 wobei R₁ ausgewählt ist aus der Gruppe bestehend aus Wasserstoff, Methyl oder Ethyl, Z ein (C₁-C₈)-Alkylen ist, das unsubstituiert oder mit einem oder mehreren (C₁-C₄)-Alkylresten substituiert sein kann, und X⁺ ausgewählt ist aus der Gruppe bestehend aus H⁺, einem Alkalimetallion, einem Erdalkalimetallion, einem Ammoniumion, einem Alkanolammoniumion, oder Mischungen davon. Vorzugsweise wird X⁺ dabei ausgewählt aus der Gruppe bestehend aus H⁺, Na⁺, NH₄⁺, oder Mischungen davon.

In einer besonders bevorzugten Ausführungsform der Erfindung ist die Acrylamidoalkylsulfonsäure bzw. das ionische Monomer (M1) 2-Acrylamido-2-methyl-propansulfonsäure (AMPS, 2-Methyl-2-[(1-oxo-2-propenyl)amino]-1-propansulfonsäure), die die chemische Formel (2) besitzt, 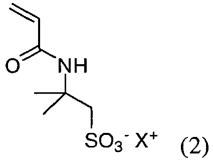 und als freie Säure (X⁺ ist gleich H⁺), oder partiell oder vollständig neutralisiert in Form ihrer Salze (der Acryloyldimethyltaurate, X⁺ ist ein Kation außer H⁺, z.B. ein Alkalimetallion wie Na⁺, ein Erdalkalimetallion, wie (1/2) Ca²⁺, oder ein Ammoniumion, wie NH₄⁺) vorliegen kann. Vorzugsweise wird X⁺ dabei ausgewählt aus der Gruppe bestehend aus H⁺, Na⁺, NH₄⁺, oder Mischungen davon. Besonders bevorzugt ist das ionische Monomer (M1) Natriumacryloyldimethyltaurat oder Ammoniumacryloyldimethyltaurat (X⁺ ist gleich Na⁺ bzw. NH₄⁺).

### Weiteres Monomer:

In einer Ausführungsform umfasst das mindestens eine weitere Monomer mindestens ein nicht-ionisches Monomer, bevorzugt ausgewählt aus der Gruppe bestehend aus Styrolen, Chlorstyrolen, Di-(C₁-C₃₀)-Alkylaminostyrolen, Vinylchloriden, Isoprenen, Vinylalkoholen, Vinylmethylethern, (C₁-C₃₀)-Carbonsäurevinylestern, bevorzugt Vinylacetaten und Vinylpropionaten; Acrylsäureestern, Methacrylsäureestern, Maleinsäureestern, Fumarsäureestern, Crotonsäureestern; insbesondere lineare und verzweigte (C₁-C₃₀)-Alkylester der Acrylsäure, Methacrylsäure, Maleinsäure, Fumarsäure und Crotonsäure; lineare und verzweigte (C₁-C₃₀)-Hydroxyalkylester der Acrylsäure, Methacrylsäure, Maleinsäure, Fumarsäure und Crotonsäure; ethoxylierte (C₁-C₃₀)-Alkylester der Acrylsäure, Methacrylsäure, Maleinsäure, Fumarsäure und Crotonsäure mit von 1 bis 40 Ethylenoxideinheiten; Acrylamiden, insbesondere N,N-Di-(C₁-C₃₀)-Alkylacrylamiden, Methacrylamiden, insbesondere N,N-Di-(C₁-C₃₀)-Alkylmethacrylamiden, cyclischen und linearen N-Vinylcarbonsäureamiden mit einer Kohlenstoffkette von 2 bis 9 Kohlenstoffatomen, bevorzugt N-Vinylpyrrolidon; und Mischungen davon.

Das mindestens eine weitere Monomer kann auch mindestens ein ionisches Monomer umfassen, bevorzugt ausgewählt aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze oder Ammoniumsalze; oder als Anhydrid vorliegen können, und Mischungen davon. In einer bevorzugten Ausführungsform umfasst das mindestens eine weitere Monomer eine Acrylsäure, die partiell oder vollständig neutralisiert in Form ihres Alkalimetallsalzes, Erdalkalimetallsalzes oder Ammoniumsalzes vorliegt. Besonders bevorzugt umfasst das mindestens eine weitere Monomer dabei Natriumacrylat.

Ein besonders geeignetes Emulgator-Copolymer ist beispielsweise Natriumacrylat/ Natriumacryloyldimethyltaurat-Copolymer, insbesondere in Form des unter dem Namen 8885MP2 (Sepinov EG-P) von der Firma Seppic erhältlichen Produkts. Ein weiteres besonders geeignetes Emulgator-Copolymer ist Natriumacrylat/ Acryloyldimethyltaurat/ Dimethylacrylamid-Crosspolymer, insbesondere in Form des unter dem Namen 8732MP (Sepinov P88) von der Firma Seppic erhältlichen Produkts. Ein weiteres besonders geeignetes Emulgator-Copolymer ist Hydroxyethylacrylat/ Natriumacryloyldimethyltaurat-Copolymer, insbesondere in Form des unter dem Handelsnamen Sepinov^{™} EMT 10 von der Firma Seppic vertriebenen Produkts.

Ein besonders geeignetes Emulgator-Copolymer ist Acryloyldimethyltaurat/ Vinylpyrrolidon-Copolymer, bevorzugt Ammoniumacryloyldimethyltaurat/ Vinylpyrrolidon-Copolymer, insbesondere in Form des unter dem Handelsnamen Aristoflex® AVC vertriebenen Produkts.

Acryloyldimethyltaurat/ Vinylpyrrolidon-Copolymer hat vorzugsweise die allgemeine Formel (3) 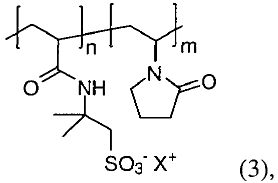 wobei X⁺ Na⁺ oder NH₄⁺ ist und n und m ganze Zahlen sind, die unabhängig voneinander zwischen 1 bis 10.000 variieren. Vorzugsweise liegt das Polymer dabei als ein statistisches Copolymer, ein Block-Copolymer oder ein Propf-Copolymer, besonders bevorzugt als ein statistisches Copolymer vor.

Bevorzugt werden die Emulgator-Copolymere in präneutralisierter Form verwendet, wobei sie vorzugsweise pulverförmig vorliegen.

In besonderen Ausführungsformen der vorliegenden Erfindung beträgt das Gewichts-Verhältnis des ionischen Monomers (M1) zu dem mindestens einen weiteren Monomer von 99:1 bis 1:99, bevorzugt von 95:5 bis 5:95, besonders bevorzugt von 90:10 bis 10:90.

Das Emulgator-Copolymer kann beispielsweise als statistisches Copolymer, Block-Copolymer oder Propf-Copolymer oder Mischungen davon vorliegen, wobei statistische Copolymere bevorzugt sind.

In speziellen Ausführungsformen der vorliegenden Erfindung ist das Emulgator-Copolymer quervernetzt, wobei das quervernetzte Emulgator-Copolymer vorzugsweise von 0,001 bis 10 Gew.%, besonders bevorzugt 0,01 bis 10 Gew.% Vernetzungsmittel enthält.

Als Vernetzungsmittel können beispielsweise Diallyloxyessigsäure oder deren Salze, Trimethylolpropantriacrylat, Trimethylolpropandiallylether, Ethylenglykoldimethacrylat, Diethylenglykoldiacrylat, Tetraethylenglykoldiacrylat, Methylenbis(acrylamid), Divinylbenzol, Diallylharnstoff, Triallylamin, 1,1,2,2-Tetraallyloxyethan, Acrylsäureallylester, Methacrylsäureallylester, Dipropylenglykoldiallylether, Polyglykoldiallylether, Triethylenglykoldivinylether, oder Hydrochinondiallylether verwendet werden.

### Feststoffemulgatoren:

Die Emulsion enthält mindestens einen Feststoffemulgator, vorzugsweise in einer Menge von mehr als 0,5 Gew.% , besonders bevorzugt mehr als 1 Gew.%. Insbesondere enthält die Emulsion von 0,5 bis 7 Gew.%, bevorzugt von 0,5 bis 5 Gew.%, besonders bevorzugt von 0,5 bis 3 Gew.% des mindestens einen Feststoffemulgators. Die Angaben werden jeweils auf das Gesamtgewicht der Emulsion ohne Treibgas bezogen.

Das Gewichts-Verhältnis des mindestens einen Feststoffemulgators zu dem mindestens einen ionischen, grenzflächenaktiven Polymer in der Emulsion beträgt bevorzugt von 0,5:1 bis 10:1, besonders bevorzugt von 2:1 bis 8:1.

Geeignete Feststoffemulgatoren sind partikuläre anorganische oder organische Feststoffe, die sowohl von lipophilen als auch von hydrophilen Flüssigkeiten benetzbar sind. Geeignete Vertreter sind z.B. Titandioxid, insbesondere beschichtetes Titanoxid (z.B. erhältlich von Merck KGaA unter der Bezeichnung Eusolex® T-2000), Zinkoxid (z.B. erhältlich von BASF AG unter der Bezeichnung Z-Cote Max), Siliziumdioxid, insbesondere hochdisperses Siliziumdioxid, Fe₂O₃, Veegum, Bentonit und Ethylcellulose. Ferner können Aluminiumoxid, Calciumcarbonat, Kohle, Magnesiumoxid, Magnesiumtrisilikat, kristalline Fettsäuren, kristalline Fettsäureester, kristalline Fettalkohole, Polymerlatices, z. B. Polystyrole oder Polymethacrylate, und Polymer-Pseudolatices verwendet werden. Auch Mischungen der vorgenannten Feststoffemulgatoren können verwendet werden. Bevorzugt ist der mindestens eine Feststoffemulgator ausgewählt aus der Gruppe bestehend aus kristallinen Fettsäuren, kristallinen Fettsäurealkylestern, kristallinen Fettalkoholen oder Mischungen davon.

Beispielsweise umfasst der mindestens eine Feststoffemulgator eine kristalline Fettsäure, vorzugsweise mit einer Kettenlänge von 10 bis 40 Kohlenstoffatomen. Insbesondere ist die kristalline Fettsäure dabei eine gesättigte Fettsäure, bevorzugt ausgewählt aus der Gruppe bestehend aus Myristinsäure, Palmitinsäure, Margarinsäure, Stearinsäure und Arachinsäure oder Mischungen davon.

In einer besonders bevorzugten Ausführungsform umfasst der mindestens eine Feststoffemulgator Stearinsäure. Stearinsäure ist beispielsweise erhältlich von der Fa. Cognis unter dem Namen Cutina FS 45.

Ferner kann der mindestens eine Feststoffemulgator einen kristallinen Fettalkohol, bevorzugt mit einer Kettenlänge von 10 bis 40 Kohlenstoffatomen umfassen. Insbesondere ist der kristalline Fettalkohol dabei ein gesättigter Fettalkohol, bevorzugt ausgewählt aus der Gruppe bestehend aus Myristylalkohol, Cetylalkohol, Heptadecanol, Stearylalkohol, Cetylstearylalkohol, Eicosanol, oder Mischungen davon.

In einer besonders bevorzugten Ausführungsform umfasst der mindestens eine Feststoffemulgator Cetylstearylalkohol. Der Cetylstearylalkohol ist beispielsweise erhältlich von der Fa. Cognis unter dem Namen Lanette O.

Ferner kann der mindestens eine Feststoffemulgator einen kristallinen Fettsäurealkylester, bevorzugt Cetylpalmitat, umfassen. Das Cetylpalmitat ist beispielsweise erhältlich von der Fa. Cognis unter dem Namen Cutina CP.

### Ölphase:

Geeignete Komponenten, welche die Ölphase bilden können, können aus den polaren und unpolaren Ölen oder deren Mischungen gewählt werden.

Die Ölphase der erfindungsgemäßen Formulierungen wird vorteilhaft gewählt aus der Gruppe der Phospholipide, wie etwa Lecithine und der Fettsäuretriglyceride, aus der Gruppe der Propylenglykol- oder Butylenglykol-Fettsäureester, aus der Gruppe der natürlichen Wachse, tierischen und pflanzlichen Ursprungs, aus der Gruppe der Esteröle, aus der Gruppe der Dialkylether und Dialkylcarbonate, aus der Gruppe der verzweigten und unverzweigten Kohlenwasserstoffe und Wachse sowie aus der Gruppe der zyklischen und linearen Silikonöle.

In einer Ausführungsform umfasst die Ölphase mindestens einen Fettsäurealkylester wie beispielsweise Ölsäuredecylester (Decyloleat) oder Cetearylisononanoat, und/oder mindestens einen Fettalkohol wie beispielsweise 2-Octyldodecanol. Ferner kann die Ölphase gesättigte aliphatische Kohlenwasserstoffe wie Paraffin enthalten.

Decyloleat ist beispielsweise von der Fa. Cognis unter dem Namen Cetiol V erhältlich. Cetearylisononanoat ist beispielsweise von der Fa. Cognis unter dem Namen Cetiol SN erhältlich. 2-Octyldodecanol ist beispielsweise von der Fa. Cognis unter dem Namen Eutanol G erhältlich.

In einer bevorzugten Ausführungsform umfasst die Ölphase mindestens ein Triglycerid.

Bevorzugt umfasst das mindestens eine Triglycerid Caprylsäure-/Caprinsäuretriglycerid erhältlich unter der Bezeichnung Miglyol 812 der Firma Sasol, und dessen Abmischung mit weiteren Öl- und Wachskomponenten.

Besonders bevorzugt sind weiterhin Triglyceride, insbesondere Caprylsäure-/Caprinsäuretriglycerid, erhältlich unter der Bezeichnung Miglyol 812 der Firma Sasol/Myritol 312 der Fa. Cognis.

Die erfindungsgemäßen Emulsionen enthalten bevorzugt von 5 bis 50 Gew.-% Ölphase, besonders bevorzugt 10 bis 35 Gew.-% und ganz besonders bevorzugt 12 bis 25 Gew.-% Ölphase. Die Angaben werden jeweils auf das Gesamtgewicht der Emulsion ohne Treibgas bezogen.

### Wasserphase:

Die Wasserphase kann kosmetische Hilfsstoffe enthalten, z.B. niedere Alkohole (z.B. Ethanol, Isopropanol), niedere Diole oder Polyole sowie deren Ether (z.B. Propylenglykol, Glycerin, Butylenglykol, Hexylenglykol und Ethylenglykol), Schaumstabilisatoren und Verdickungsmittel.

Geeignete Verdickungsmittel sind polymere Verdickungsmittel, die teilweise wasserlöslich oder zumindest in Wasser dispergierbar sind und in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie die Beweglichkeit des Wassers einschränken. Geeignete Polymere sind:
- Abgewandelte Naturstoffe, wie Celluloseether (z.B. Hydroxypropylcelluloseether, Hydroxyethylcellulose und Hydroxypropylmethylcelluloseether);
- Natürliche Verbindungen, wie z.B. Xanthan, Agar-Agar, Carrageen, Polyosen, Stärke, Dextrine, Gelatine, Casein;
- Synthetische Verbindungen wie z.B. Vinylpolymere, Polyether, Polyimine, Polyamide und Derivate der Polyacrylsäure; und
- Anorganische Verbindungen wie z.B. Polykieselsäure und Tonmineralien.

Bevorzugt enthält die Emulsion mindestens ein Verdickungsmittel ausgewählt aus der Gruppe bestehend aus Hydroxypropylmethylcellulose, Xanthan Gum, Natriumpolyacrylat, und Mischungen davon.

Eine erfindungsgemäße, bevorzugte Hydroxypropylmethylcellulose ist Metolose 90SH 100. Die allgemeine Arzneibuchbezeichnung für Hydroxypropylmethylcellulose ist Hypromellose.

Xanthan Gummi ist beispielsweise von der Fa. Kelco unter dem Namen Keltrol® CG erhältlich. Natriumpolyacrylat ist beispielsweise von der Fa. Cognis unter dem Namen Cosmedia SP erhältlich.

Die erfindungsgemäßen Emulsionen enthalten bevorzugt von 0,2 bis 1,5 Gew.-% Verdickungsmittel (bezogen auf das Trockengewicht des Verdickungsmittels und das Gesamtgewicht der Emulsion ohne Treibgas). Besonders bevorzugt sind 0,2 bis 0,8 Gew.-% Verdickungsmittel.

### Wirkstoffe:

Der gegebenenfalls enthaltene Wirkstoff kann unter allen oberflächig auf der Haut applizierbaren Wirkstoffen und Mischungen dieser gewählt werden. Der Wirkstoff kann kosmetisch oder pharmazeutisch wirken. Entsprechend erhält man kosmetische oder dermatologische (als Medizinprodukt oder Arzneimittelprodukt einzusetzende) Schaumformulierungen. Ferner kann die Formulierung zum Schutz der Haut vor Umwelteinflüssen dienen. Der Wirkstoff kann natürlich oder synthetisch sein. Die Gruppe der Wirkstoffe kann sich auch mit den anderen Inhaltstoffgruppen, wie z.B. der Ölkomponente, den Verdickungsmitteln oder den Feststoffemulgatoren, überschneiden. Beispielsweise können manche Ölkomponenten auch als Wirkstoffe dienen, wie z.B. Öle mit mehrfach ungesättigten Fettsäuren, oder Feststoffemulgatoren, wie z.B. partikuläres Titandioxid als UV-Filter dienen können. Je nach Eigenschaftsprofil sind die Substanzen mehreren Gruppen zuzuordnen.

Wirkstoffe der erfindungsgemäßen Formulierungen werden vorteilhaft gewählt aus der Gruppe der Substanzen mit feuchtigkeitspendenden und barrierestärkenden Eigenschaften, wie z. B. Hydroviton, eine Nachbildung des NMF, Pyrrolidoncarbonsäure und deren Salze, Milchsäure und deren Salze, Glycerol, Sorbitol, Propylenglykol und Harnstoff, Substanzen aus der Gruppe der Proteine und Proteinhydrolysate wie z. B. Collagen, Elastin sowie Seidenprotein, Substanzen aus der Gruppe der Glucosaminoglucane, wie z. B. Hyaluronsäure, aus der Gruppe der Kohlenhydrate, wie z. B. Pentavitin, das in seiner Zusammensetzung dem Kohlehydratgemisch der menschlichen Hornschicht entspricht, und der Gruppe der Lipide und Lipidvorstufen wie beispielsweise die Ceramide. Weitere vorteilhafte Wirkstoffe können im Sinne der vorliegenden Erfindung ferner gewählt werden aus der Gruppe der Vitamine, wie z. B. Panthenol, Niacin, α-Tocopherol und seine Ester, Vitamin A sowie Vitamin C. Außerdem können die Wirkstoffe, gewählt aus der Gruppe der Antioxidantien z. B. Galate und Polyphenole, verwendet werden. Harnstoff, Hyaluronsäure und Pentavitin sind bevorzugte Substanzen.

Es ist ferner bevorzugt, dass als Wirkstoffe Substanzen mit hautberuhigender und regenerierender Wirkung eingesetzt werden, wie z. B. Panthenol, Bisabolol und Phytosterole.

In einer bevorzugten Ausführungsform enthält die erfindungsgemäße Schaumformulierung Harnstoff. In einer weiteren bevorzugten Ausführungsform enthält die erfindungsgemäße Schaumformulierung keine α- oder β-Hydroxycarbonsäuren oder deren Salze, insbesondere keine C₁-C₂₅-α- oder C₁-C₂₅-β-Hydroxycarbonsäuren oder deren Salze.

Vorteilhafte Wirkstoffe im Sinne der vorliegenden Erfindung sind auch Pflanzen und Pflanzenextrakte. Hierzu gehören z.B. Algen, Aloe, Arnika, Bartflechten, Beinwell, Birke, Brennessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss sowie deren Extrakte.

Die erfindungsgemäßen Zubereitungen können ferner als Wirkstoffe Antimykotika und Antiseptika/Desinfizientia synthetischen oder natürlichen Ursprungs enthalten.

Weitere Wirkstoffe sind Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, wundheilungsfördernde Wirkstoffe, z.B. Wachstumsfaktoren, Enzympräparate und Insektizide.

### Weitere Bestandteile der Emulsion:

Die Formulierungen können außerdem optional Farbstoffe, Perlglanzpigmente, Duftstoffe/Parfum, Lichtschutzfiltersubstanzen, Konservierungsmittel, Komplexbildner, Antioxidantien und Repellentien sowie pH-Wert Regulatoren enthalten. In einer bevorzugten Ausführungsform sind die Formulierungen der Erfindung jedoch frei von Bestandteilen, die zu Irritationen der Haut führen können, insbesondere frei von Duftstoffen/Parfum, Farbstoffen und herkömmlichen Emulgatoren.

Die erfindungsgemäßen Schaumformulierungen können neben den bereits oben genannten Bestandteilen weitere natürliche Fette, wie z.B. Sheabutter, Neutralöle, Olivenöl, Squalan, Ceramide und Feuchthaltesubstanzen enthalten, wie sie in der Technik üblich sind.

Die obige Aufzählung der einzelnen Bestandteile der Emulsion soll so verstanden werden, dass einzelne Beispielkomponenten aufgrund ihrer verschiedenen Eigenschaften auch mehreren Gruppen zugeordnet werden können.

### Treibgase:

Geeignete Treibgase sind z. B. N₂O, Propan, Butan und i-Butan. Die fertige Schaumformulierung enthält beispielsweise von 1 bis 20 Gew.%, von 2 bis 18 Gew.% oder von 5 bis 15 Gew.-%, bevorzugt etwa 10 Gew.-% Treibgas. Bei der Beaufschlagung der Emulsion mit Treibgas wird (druck)verflüssigtes Treibgas verwendet.

### 3. Herstellungsverfahren

Die erfindungsgemäßen Schaumformulierungen werden durch Bereitstellen einer Emulsion vom Typ Öl in Wasser und Abfüllen dieser Emulsion und gegebenenfalls Beaufschlagen mit Treibgas in einen geeigneten Behälter, vorzugsweise in einen Druckbehälter, hergestellt. Alternativ zum Treibgas und Druckbehälter kann die Polymer-stabilisierte Emulsion auch in einen anderen Behälter abgefüllt werden, der auch ohne Treibgas dafür geeignet ist, die Emulsion als Schaum abzugeben. Solche Systeme sind dem Fachmann bekannt.

Insbesondere umfasst das Verfahren zur Herstellung der Emulsion folgende Schritte:
(1) Bereitstellen einer liquiden Ölphase enthaltend mindestens einen Feststoffemulgator,
(2) Bereitstellen einer Wasserphase enthaltend mindestens ein grenzflächenaktives, ionisches Polymer mit einem Molekulargewicht von größer als 5000 g/mol, wobei das ionische Polymer ein Copolymer ist, das als Monomereinheiten
   - ein ionisches Monomer (M1), und
   - mindestens ein weiteres Monomer
   enthält,
(3) Mischen und Homogenisieren der Wasserphase mit der Ölphase.

In einer bevorzugten Ausführungsform wird die liquide Ölphase in Schritt (1) in Form einer klaren Schmelze bereitgestellt, vorzugsweise durch Erwärmen auf eine Temperatur von 60 bis 90 °C, besonders bevorzugt 60 bis 80 °C, ganz besonders bevorzugt ungefähr 70 °C, und gegebenenfalls anschließend auf die in Schritt (3) verwendete Temperatur abgekühlt.

Bevorzugt erfolgt das Mischen und Homogenisieren der Wasserphase mit der Ölphase in Schritt (3) bei einer Temperatur von 25 bis 60°C, mehr bevorzugt von 30 bis 50°C, besonders bevorzugt von 35 bis 45 °C, und ganz besonders bevorzugt bei ungefähr 40°C.

Wenn die Polymer-stabilisierte Emulsion mindestens ein Verdickungsmittel umfasst, so enthält vorzugsweise die in Schritt (1) bereitgestellte Ölphase mindestens ein Verdickungsmittel, und/oder enthält vorzugsweise die in Schritt (2) bereitgestellte Wasserphase mindestens ein Verdickungsmittel, und/oder umfasst das Verfahren vorzugsweise die folgenden weiteren Schritte:
(4) Bereitstellen einer wässrigen Verdickungsmittellösung,
(5) Mischen der Verdickungsmittellösung mit der in Schritt (3) erhaltenen Emulsion.

Gemäß einer weiteren Ausführungsform der vorliegenden Erfindung umfasst das Verfahren zur Herstellung der Polymer-stabilisierten Emulsion folgende Schritte:
(1) Bereitstellen einer liquiden Ölphase enthaltend mindestens einen Feststoffemulgator,
(2) Bereitstellen einer Wasserphase,
(3) Mischen und Homogenisieren der Wasserphase mit der Ölphase, um eine Emulsion zu erhalten,
(4) Bereitstellen einer weiteren Wasserphase enthaltend mindestens ein grenzflächenaktives, ionisches Polymer mit einem Molekulargewicht von größer als 5000 g/mol, wobei das ionische Polymer ein Copolymer ist, das als Monomereinheiten
   - ein ionisches Monomer (M1), und
   - mindestens ein weiteres Monomer
   enthält,
(5) Mischen der weiteren Wasserphase mit der in Schritt (3) erhaltenen Emulsion.

In einer bevorzugten Ausführungsform wird die liquide Ölphase in Schritt (1) in Form einer klaren Schmelze bereitgestellt, vorzugsweise durch Erwärmen auf eine Temperatur von 60 bis 90 °C, besonders bevorzugt 60 bis 80 °C, ganz besonders bevorzugt ungefähr 70 °C, und gegebenenfalls anschließend auf die in Schritt (3) verwendete Temperatur abgekühlt.

Bevorzugt erfolgt das Mischen und Homogenisieren der Wasserphase mit der Ölphase in Schritt (3) bei einer Temperatur von 25 bis 60°C, mehr bevorzugt von 30 bis 50°C, besonders bevorzugt von 35 bis 45 °C, und ganz besonders bevorzugt bei ungefähr 40°C.

In einer bevorzugten Ausführungsform erfolgt das Mischen der weiteren Wasserphase mit der Emulsion in Schritt (5) bei einer Temperatur von 10 bis 30°C, bevorzugt von 15 bis 25°C und besonders bevorzugt bei Raumtemperatur.

Wenn die Polymer-stabilisierte Emulsion mindestens ein Verdickungsmittel umfasst, so enthält vorzugsweise die in Schritt (1) bereitgestellte Ölphase mindestens ein Verdickungsmittel, und/oder enthält vorzugsweise die in Schritt (2) bereitgestellte Wasserphase mindestens ein Verdickungsmittel, und/oder enthält vorzugsweise die in Schritt (4) bereitgestellte weitere Wasserphase mindestens ein Verdickungsmittel, und/oder umfasst das Verfahren vorzugsweise die folgenden weiteren Schritte:
(6) Bereitstellen einer wässrigen Verdickungsmittellösung,
(7) Mischen der Verdickungsmittellösung mit der in Schritt (5) erhaltenen Emulsion.

Für den Fachmann ist ersichtlich, dass zur Herstellung der erfindungsgemäß eingesetzten, Polymer-stabilisierten Emulsionen auch Kombinationen der oben genannten Herstellungsverfahren möglich sind.

Die gemäß den obigen Verfahren hergestellten Emulsionen werden zur Herstellung der Schaumformulierung bevorzugt mit 1 bis 20 Gew.%, bevorzugt 2 bis 18 Gew.%, mehr bevorzugt 5 bis 15 Gew.%, ganz besonders bevorzugt 10 Gew.-% Treibgas bezogen auf das Gewicht der Schaumformulierung beaufschlagt. Bei dem Treibgas handelt es sich vorzugsweise um druckverflüssigtes Treibgas.

### 4. Verwendungen

Die erfindungsgemäßen Schaumformulierungen können für alle kosmetischen und dermatologischen (als Medizinprodukt oder Arzneimittel) Zwecke eingesetzt werden. Zum Beispiel können die Schaumformulierungen als Hautpflegemittel oder Hautreinigungsmittel eingesetzt werden. Sie können ferner als Träger für Wirkstoffe dienen und im medizinisch dermatologischen Bereich eingesetzt werden. Insbesondere können die Formulierungen als Sonnenschutzmittel eingesetzt werden. Viele der Feststoffemulgatoren, wie beispielsweise Titandioxid, sind wirksame UVA und UVB Filter.

### 5. Bevorzugte Ausführungsformen

Die vorliegende Erfindung ist insbesondere auf die folgenden bevorzugten Ausführungsformen gerichtet:
1. Schaumformulierung gemäß Anspruch 2 umfassend, eine im Wesentlichen emulgatorfreie Emulsion vom Typ Öl in Wasser, umfassend eine Ölphase und eine Wasserphase, wobei die Emulsion umfasst:
   a) mindestens einen Feststoffemulgator, und
   b) mindestens ein grenzflächenaktives, ionisches Polymer mit einem Molekulargewicht von größer als 5000 g/mol, das die Emulsion durch Ausbildung einer Gelstruktur an der Öl-/Wasser-Phasengrenzfläche stabilisiert, wobei das ionische Polymer ein Copolymer ist, das als Monomereinheiten
      - ein ionisches Monomer (M1), und
      - mindestens ein weiteres Monomer
      enthält.
2. Schaumformulierung gemäß der Ausführungsform 1, wobei das ionische Polymer anionisch, kationisch oder zwitterionisch, bevorzugt anionisch ist.
3. Schaumformulierung gemäß einer Ausführungsform 1 oder 2, wobei die Gelstruktur an der Öl-/Wasser-Phasengrenzfläche in Form einer die Ölphase umgebenden Schicht aus Geltröpfchen vorliegt.
4. Schaumformulierung gemäß Ausführungsform 3, wobei die Geltröpfchen stark hydratisiert sind.
5. Schaumformulierung gemäß einer der Ausführungsformen 1 bis 4, wobei das mindestens eine grenzflächenaktive, ionische Polymer zusätzlich als Verdickungsmittel wirkt.
6. Schaumformulierung gemäß einer der Ausführungsformen 1 bis 5, wobei die Gelstruktur an der Öl-/Wasser-Phasengrenzfläche eine höhere Viskosität aufweist als die die Gelstruktur umgebende Wasserphase.
7. Schaumformulierung gemäß einer der Ausführungsformen 1 bis 6, wobei die Emulsion weniger als 0,3 Gew.-%, mehr bevorzugt weniger als 0,1 Gew.-% herkömmlicher Emulgatoren enthält.
8. Schaumformulierung gemäß einer der Ausführungsformen 1 bis 7, wobei die Emulsion keine herkömmlichen Emulgatoren enthält.
9. Schaumformulierung gemäß einer der Ausführungsformen 1 bis 8, wobei das mindestens eine grenzflächenaktive, ionische Polymer wasserlöslich oder wasserquellbar, bevorzugt wasserquellbar, ist.
10. Schaumformulierung gemäß einer der Ausführungsformen 1 bis 9, wobei die Emulsion von ungefähr 0,01 bis ungefähr 10 Gew.%, bevorzugt von ungefähr 0,05 bis ungefähr 8 Gew.%, mehr bevorzugt von ungefähr 0,1 bis ungefähr 5 Gew.%, ganz besonders bevorzugt von ungefähr 0,2 bis ungefähr 1 Gew.%. des mindestens einen grenzflächenaktiven, ionischen Polymers bezogen auf das Gesamtgewicht der Emulsion (ohne Treibgas) enthält.
11. Schaumformulierung gemäß einer der Ausführungsformen 1 bis 10, wobei das mindestens eine weitere Monomer eine andere Polarität besitzt als das ionische Monomer (M1).
12. Schaumformulierung gemäß einer der Ausführungsformen 1 bis 11, wobei das mindestens eine weitere Monomer ausgewählt ist aus der Gruppe bestehend aus ionischen Monomeren, nicht-ionischen Monomeren, und Mischungen davon.
13. Schaumformulierung gemäß einer der Ausführungsformen 1 bis 12, wobei das mindestens eine weitere Monomer mindestens ein nicht-ionisches Monomer umfasst.
14. Schaumformulierung gemäß einer der Ausführungsformen 1 bis 13, wobei das ionische Monomer (M1) freie, partiell neutralisierte oder vollständig neutralisierte saure funktionelle Gruppen enthält.
15. Schaumformulierung gemäß Ausführungsform 14,
   wobei die sauren funktionellen Gruppen ausgewählt sind aus der Gruppe bestehend aus Sulfonsäuregruppen, Carbonsäuregruppen, Phosphorsäuregruppen, Phosphonsäuregruppen und Mischungen davon.
16. Schaumformulierung gemäß einer der Ausführungsformen 1 bis 15, wobei das ionische Monomer (M1) ausgewählt ist aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze, Ammoniumsalze oder Alkanolammoniumsalze; oder als Anhydrid vorliegen können, und Mischungen davon.
17. Schaumformulierung gemäß einer der Ausführungsformen 1 bis 16, wobei das ionische Monomer (M1) eine Acrylamidoalkylsulfonsäure ist, die als freie Säure, partiell oder vollständig neutralisiert in Form ihrer Salze vorliegt.
18. Schaumformulierung gemäß Ausführungsform 17,
   wobei die Acrylamidoalkylsulfonsäure partiell oder vollständig neutralisiert ist und als Alkalimetall-, Erdalkalimetall-, Ammonium- oder Alkanolammoniumsalz, bevorzugt als Natrium- oder Ammoniumsalz, besonders bevorzugt als Ammoniumsalz vorliegt.
19. Schaumformulierung gemäß einer der Ausführungsformen 17 oder 18, wobei die Acrylamidoalkylsulfonsäure 2-Acrylamido-2-methyl-propansulfonsäure ist.
20. Schaumformulierung gemäß einer der Ausführungsformen 2 bis 18, wobei das ionische Monomer (M1) eine Acrylamidoalkylsulfonsäure der allgemeinen Formel (1) ist, 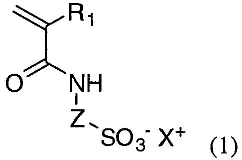 wobei R₁ ausgewählt ist aus der Gruppe bestehend aus Wasserstoff, Methyl oder Ethyl, Z ein (C₁-C₈)-Alkylen ist, das unsubstituiert oder mit einem oder mehreren (C₁-C₄)-Alkylresten substituiert sein kann, und X⁺ ausgewählt ist aus der Gruppe bestehend aus H⁺, einem Alkalimetallion, einem Erdalkalimetallion, einem Ammoniumion, einem Alkanolammoniumion, oder Mischungen davon, vorzugsweise aus der Gruppe bestehend aus H⁺, Na⁺, NH₄⁺ oder Mischungen davon.
21. Schaumformulierung gemäß einer der Ausführungsformen 1 bis 18, wobei das ionische Monomer (M1) 2-Acrylamido-2-methyl-propansulfonsäure der allgemeinen Formel (2) ist 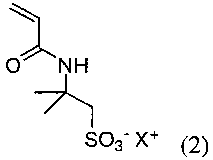 wobei X⁺ ausgewählt ist aus der Gruppe bestehend aus H⁺, einem Alkalimetallion, einem Erdalkalimetallion, einem Ammoniumion, einem Alkanolammoniumion, oder Mischungen davon, vorzugsweise aus der Gruppe bestehend aus H⁺, Na⁺, NH₄⁺ oder Mischungen davon.
22. Schaumformulierung gemäß einer der Ausführungsformen 1 bis 21, wobei das ionische Monomer (M1) Natriumacryloyldimethyltaurat oder Ammoniumacryloyldimethyltaurat ist.
23. Schaumformulierung gemäß einer der Ausführungsformen 1 bis 22, wobei das mindestens eine weitere Monomer mindestens ein nicht-ionisches Monomer umfasst ausgewählt aus der Gruppe bestehend aus Styrolen, Chlorstyrolen, Di-(C₁-C₃₀)-Alkylaminostyrolen, Vinylchloriden, Isoprenen, Vinylalkoholen, Vinylmethylethern, (C₁-C₃₀)-Carbonsäurevinylestern, bevorzugt Vinylacetaten und Vinylpropionaten; Acrylsäureestern, Methacrylsäureestern, Maleinsäureestern, Fumarsäureestern, Crotonsäureestern; insbesondere lineare und verzweigte (C₁-C₃₀)-Alkylester der Acrylsäure, Methacrylsäure, Maleinsäure, Fumarsäure und Crotonsäure; lineare und verzweigte (C₁-C₃₀)-Hydroxyalkylester der Acrylsäure, Methacrylsäure, Maleinsäure, Fumarsäure und Crotonsäure; ethoxylierte (C₁-C₃₀)-Alkylester der Acrylsäure, Methacrylsäure, Maleinsäure, Fumarsäure und Crotonsäure mit von 1 bis 40 Ethylenoxideinheiten; Acrylamiden, insbesondere N,N-Di-(C₁-C₃₀)-Alkylacrylamiden, Methacrylamiden, insbesondere N,N-Di-(C₁-C₃₀)-Alkylmethacrylamiden, cyclischen und linearen N-Vinylcarbonsäureamiden mit einer Kohlenstoffkette von 2 bis 9 Kohlenstoffatomen, bevorzugt N-Vinylpyrrolidon; und Mischungen davon.
24. Schaumformulierung gemäß einer der Ausführungsformen 1 bis 23, wobei das mindestens eine weitere Monomer mindestens ein nicht-ionisches Monomer umfasst ausgewählt aus der Gruppe bestehend aus linearen und verzweigten (C₁-C₃₀)-Alkylestern der Acrylsäure oder Methacrylsäure; linearen und verzweigten (C₁-C₃₀)-Hydroxyalkylestern der Acrylsäure oder Methacrylsäure; ethoxylierten (C₁-C₃₀)-Alkylestern der Acrylsäure oder Methacrylsäure mit von 1 bis 40 Ethylenoxideinheiten; N,N-Di-(C₁-C₃₀)-Alkylacrylamiden, N,N-Di-(C₁-C₃₀)-Alkylmethacrylamiden, cyclischen und linearen N-Vinylcarbonsäureamiden mit einer Kohlenstoffkette von 2 bis 9 Kohlenstoffatomen, bevorzugt N-Vinylpyrrolidon; und Mischungen davon.
25. Schaumformulierung gemäß einer der Ausführungsformen 1 bis 24, wobei das mindestens eine weitere Monomer mindestens ein nicht-ionisches Monomer umfasst ausgewählt aus der Gruppe bestehend aus linearen und verzweigten (C₁-C₆)-Hydroxyalkylestern der Acrylsäure oder Methacrylsäure, bevorzugt Hydroxyethylacrylat; ethoxylierten (C₁-C₃₀)-Alkylestern der Acrylsäure oder Methacrylsäure mit von 1 bis 40 Ethylenoxideinheiten, bevorzugt Beheneth-25-Methacrylat; N,N-Di-(C₁-C₆)-Alkylacrylamiden, bevorzugt N,N-Dimethylacrylamid, cyclischen und linearen N-Vinylcarbonsäureamiden mit einer Kohlenstoffkette von 2 bis 9 Kohlenstoffatomen, bevorzugt N-Vinylpyrrolidon, und Mischungen davon.
26. Schaumformulierung gemäß einer der Ausführungsformen 1 bis 25, wobei das mindestens eine weitere Monomer mindestens ein ionisches Monomer umfasst ausgewählt aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze oder Ammoniumsalze; oder als Anhydrid vorliegen können, und Mischungen davon.
27. Schaumformulierung gemäß Ausführungsform 26,
   wobei das mindestens eine weitere Monomer eine Acrylsäure umfasst, die partiell oder vollständig neutralisiert in Form ihres Alkalimetallsalzes, Erdalkalimetallsalzes oder Ammoniumsalzes vorliegt.
28. Schaumformulierung gemäß einer der Ausführungsformen 26 oder 27, wobei das mindestens eine weitere Monomer Natriumacrylat umfasst.
29. Schaumformulierung gemäß einer der Ausführungsformen 1 bis 28, wobei das mindestens eine grenzflächenaktive, ionische Polymer ausgewählt ist aus der Gruppe bestehend aus Acryloyldimethyltaurat/ Vinylpyrrolidon-Copolymer, Natriumacrylat/ Acryloyldimethyltaurat/ Dimethylacrylamid-Crosspolymer, Hydroxyethylacrylat/ Natriumacryloyldimethyltaurat-Copolymer, Natriumacrylat/ Natriumacryloyldimethyltaurat-Copolymer, und Mischungen davon.
30. Schaumformulierung gemäß einer der Ausführungsformen 1 bis 29, wobei das mindestens eine grenzflächenaktive, ionische Polymer Acryloyldimethyltaurat/ Vinylpyrrolidon-Copolymer, besonders bevorzugt Ammoniumacryloyldimethyltaurat/ Vinylpyrrolidon-Copolymer, ist.
31. Schaumformulierung gemäß einer der Ausführungsformen 1 bis 30, wobei das mindestens eine grenzflächenaktive, ionische Polymer Acryloyldimethyltaurat/ Vinylpyrrolidon-Copolymer mit der allgemeinen Formel (3) ist, 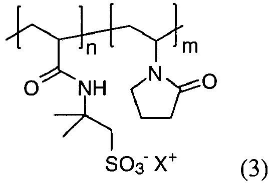 wobei X⁺ Na⁺ oder NH₄⁺, bevorzugt NH₄⁺, ist und n und m ganze Zahlen sind, die unabhängig voneinander zwischen 1 bis 10.000 variieren.
32. Schaumformulierung gemäß einer der Ausführungsformen 1 bis 29, wobei das mindestens eine grenzflächenaktive, ionische Polymer Natriumacrylat/ Acryloyldimethyltaurat/ Dimethylacrylamid-Crosspolymer ist.
33. Schaumformulierung gemäß einer der Ausführungsformen 1 bis 29, wobei das mindestens eine grenzflächenaktive, ionische Polymer Hydroxyethylacrylat/ Natriumacryloyldimethyltaurat-Copolymer ist.
34. Schaumformulierung gemäß einer der Ausführungsformen 1 bis 29, wobei das mindestens eine grenzflächenaktive, ionische Polymer Natriumacrylat/ Natriumacryloyldimethyltaurat-Copolymer ist.
35. Schaumformulierung gemäß einer der Ausführungsformen 1 bis 34, wobei das Copolymer in präneutralisierter Form verwendet wird, vorzugsweise in präneutralisierter, pulverförmiger Form.
36. Schaumformulierung gemäß einer der Ausführungsformen 1 bis 35, wobei das Gewichts-Verhältnis des ionischen Monomers (M1) zu dem mindestens einen weiteren Monomer von 99:1 bis 1:99, bevorzugt von 95:5 bis 5:95, besonders bevorzugt von 90:10 bis 10:90 beträgt.
37. Schaumformulierung gemäß einer der Ausführungsformen 1 bis 36, wobei das Copolymer quervernetzt ist.
38. Schaumformulierung gemäß Ausführungsform 37,
   wobei das Copolymer von 0,001 bis 10 Gew.%, bevorzugt von 0,01 bis 10 Gew.% Vernetzungsmittel enthält.
39. Schaumformulierung gemäß Ausführungsform 37 oder 38,
   wobei das Vernetzungsmittel ausgewählt ist aus der Gruppe bestehend aus Diallyloxyessigsäure oder deren Salzen, Trimethylolpropantriacrylat, Trimethylolpropandiallylether, Ethylenglykoldimethacrylat, Diethylenglykoldiacrylat, Tetraethylenglykoldiacrylat, Methylenbis(acrylamid), Divinylbenzol, Diallylharnstoff, Triallylamin, 1,1,2,2-Tetraallyloxyethan, Acrylsäureallylester, Methacrylsäureallylester, Dipropylenglykoldiallylether, Polyglykoldiallylether, Triethylenglykoldivinylether, Hydrochinondiallylether, oder Mischungen davon.
40. Schaumformulierung gemäß einer der Ausführungsformen 1 bis 39, wobei das Copolymer ein statistisches Copolymer, ein Block-Copolymer oder ein Propf-Copolymer, bevorzugt ein statistisches Copolymer ist.
41. Schaumformulierung gemäß einer der Ausführungsformen 1 bis 40, wobei die Emulsion mehr als 0,5 Gew.%, bevorzugt mehr als 1 Gew.% des mindestens einen Feststoffemulgators bezogen auf das Gesamtgewicht der Emulsion ohne Treibgas enthält.
42. Schaumformulierung gemäß einer der Ausführungsformen 1 bis 41, wobei die Emulsion von 0,5 bis 7 Gew.%, bevorzugt von 0,5 bis 5 Gew.%, besonders bevorzugt von 0,5 bis 3 Gew.% des mindestens einen Feststoffemulgators bezogen auf das Gesamtgewicht der Emulsion ohne Treibgas enthält.
43. Schaumformulierung gemäß einer der Ausführungsformen 1 bis 42, wobei das Gewichts-Verhältnis des mindestens einen Feststoffemulgators zu dem mindestens einen ionischen, grenzflächenaktiven Polymer in der Emulsion von 0,5:1 bis 10:1, bevorzugt von 2:1 bis 8:1 beträgt.
44. Schaumformulierung gemäß einer der Ausführungsformen 1 bis 43, wobei die Emulsion mindestens einen partikulären Feststoffemulgator, ausgewählt aus der Gruppe bestehend aus Titandioxid, Siliziumdioxid, Fe₂O₃, Zinkoxid, Veegum, Bentonit und Ethylcellulose, Aluminiumoxid, Calciumcarbonat, Kohle, Magnesiumoxid, Magnesiumtrisilikat, kristalline Fettsäuren, kristalline Fettsäureester, kristalline Fettalkohole, Polymerlatices, wie etwa Polystyrole oder Polymethacrylate, und Polymer-Pseudolatices oder Mischungen davon umfasst.
45. Schaumformulierung gemäß Ausführungsform 44,
   wobei die Emulsion mindestens einen Feststoffemulgator ausgewählt aus der Gruppe bestehend aus kristallinen Fettsäuren, kristallinen Fettsäurealkylestern, kristallinen Fettalkoholen oder Mischungen davon umfasst.
46. Schaumformulierung gemäß Ausführungsform 44 oder 45,
   wobei der mindestens eine Feststoffemulgator eine kristalline Fettsäure, bevorzugt mit einer Kettenlänge von 10 bis 40 Kohlenstoffatomen, umfasst.
47. Schaumformulierung gemäß einer der Ausführungsformen 44 bis 46, wobei die kristalline Fettsäure eine gesättigte Fettsäure ist, bevorzugt ausgewählt aus der Gruppe bestehend aus Myristinsäure, Palmitinsäure, Margarinsäure, Stearinsäure und Arachinsäure oder Mischungen davon, besonders bevorzugt Stearinsäure.
48. Schaumformulierung gemäß einer der Ausführungsformen 44 bis 47, wobei der mindestens eine Feststoffemulgator einen kristallinen Fettalkohol, bevorzugt mit einer Kettenlänge von 10 bis 40 Kohlenstoffatomen umfasst.
49. Schaumformulierung gemäß einer der Ausführungsformen 44 bis 48, wobei der kristalline Fettalkohol ein gesättigter Fettalkohol ist, bevorzugt ausgewählt aus der Gruppe bestehend aus Myristylalkohol, Cetylalkohol, Heptadecanol, Stearylalkohol, Cetylstearylalkohol, Eicosanol, oder Mischungen davon, besonders bevorzugt Cetylstearylalkohol.
50. Schaumformulierung gemäß einer der Ausführungsformen 44 bis 49, wobei der mindestens eine Feststoffemulgator einen kristallinen Fettsäurealkylester, bevorzugt Cetylpalmitat, umfasst.
51. Schaumformulierung gemäß einer der vorangehenden Ausführungsformen, wobei die Ölphase mindestens ein Triglycerid umfasst.
52. Schaumformulierung gemäß Ausführungsform 51,
   wobei das Triglycerid Caprylsäure/Caprinsäuretriglycerid umfasst.
53. Schaumformulierung gemäß einer der vorangehenden Ausführungsformen, wobei die Ölphase mindestens einen Fettsäurealkylester und/oder Fettalkohol umfasst, bevorzugt ausgewählt aus der Gruppe bestehend aus Decyloleat, Cetearylisononanoat und 2-Octyldodecanol, und Mischungen davon.
54. Schaumformulierung gemäß einer der vorangehenden Ausführungsformen, wobei die Emulsion mindestens ein Verdickungsmittel umfasst.
55. Schaumformulierung gemäß einer der vorangehenden Ausführungsformen, wobei die Emulsion von 0,2 bis 1,5 Gew.-% Verdickungsmittel, bevorzugt von 0,2 bis 0,8 Gew.-% Verdickungsmittel, bezogen auf das Trockengewicht des Verdickungsmittels und das Gesamtgewicht der Emulsion ohne Treibgas enthält.
56. Schaumformulierung gemäß Ausführungsform 54 oder 55,
   wobei das Verdickungsmittel ausgewählt ist aus der Gruppe bestehend aus Xanthan Gum, Natriumpolyacrylat, Hydroxypropylmethylcellulose und Mischungen davon.
57. Schaumformulierung gemäß einer der vorangehenden Ausführungsformen, wobei die Emulsion mindestens einen Wirkstoff enthält.
58. Schaumformulierung gemäß Ausführungsform 57,
   wobei der Wirkstoff ausgewählt ist aus der Gruppe bestehend aus Hydroviton, Pyrrolidoncarbonsäure und deren Salze, Milchsäure und deren Salze, Glycerol, Sorbitol, Propylenglykol, Harnstoff, Collagen, Elastin, Seidenprotein, Hyaluronsäure, Pentavitin, Ceramide, Panthenol, Niacin, α-Tocopherol und seine Ester, Vitamin A, Vitamin C, Galate, Polyphenole, Panthenol, Bisabolol, Phytosterole, Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, wundheilungsfördernde Wirkstoffe, Wachstumsfaktoren, Enzympräparate, Insektizide und Pflanzenmaterial wie bzw. Pflanzenextrakte von Algen, Aloe, Arnika, Bartflechten, Beinwell, Birke, Brennnessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss oder Mischungen davon.
59. Schaumformulierung gemäß einer der vorangehenden Ausführungsformen, wobei die Schaumformulierung eine Schaumcreme ist.
60. Schaumformulierung gemäß einer der vorangehenden Ausführungsformen, wobei die Schaumformulierung Treibgas, bevorzugt druckverflüssigtes Treibgas enthält.
61. Schaumformulierung gemäß Ausführungsform 60, wobei das Treibgas ausgewählt ist aus der Gruppe bestehend aus N₂O, Propan, Butan, i-Butan, und Mischungen davon.
62. Schaumformulierung gemäß einer der vorangehenden Ausführungsformen, wobei die Schaumformulierung von 1 bis 20 Gew.%, bevorzugt von 2 bis 18 Gew.%, mehr bevorzugt von 5 bis 15 Gew.% Treibgas enthält.
63. Schaumformulierung gemäß einer der vorangehenden Ausführungsformen, wobei die Schaumformulierung in einem Druckbehälter vorliegt.

### 6. Beispiele

### 6.1. Beispiele A:

| | | **Beispiel A1** | **Beispiel A2** | **Beispiel A3** | **Beispiel A4** | **Beispiel A5** | **Beispiel A6** |
|---|---|---|---|---|---|---|---|
| | | | | | | | |
| **Phase 1:** | Miglyol 812 | 5 | 5 | 5 | 5 | 5 | 5 |
| | Cetiol V | 5 | 4 | 4 | 4 | 5 | 5 |
| | Cetiol SN | 5 | 4 | 4 | 4 | 5 | 5 |
| | Eutanol G | 5 | 4 | 4 | 4 | 4 | 4 |
| | Stearinsäure | 0 | 1 | 1 | 1 | 1 | 0 |
| | Cutina CP | 0 | 2 | 2 | 2 | 0 | 0 |
| | Cetearylalkohol | 0 | 0 | 0 | 0 | 0 | 1 |
| | | | | | | | |
| **Phase 2:** | Metholose SH 100 | 0,4 | 0,4 | 0,4 | 0,4 | 0,4 | 0,4 |
| | Harnstoff | 0 | 0 | 0 | 0 | 0 | 0 |
| | Wasser | 39,6 | 39,6 | 39,6 | 39,6 | 39,6 | 39,6 |
| | | | | | | | |
| **Phase 3:** | Aristoflex HMB | 0 | 0 | 0,4 | 0 | 0 | 0 |
| | Sepinov EMT 10 | 0 | 0 | 0 | 0,4 | 0 | 0 |
| | Aristoflex AVC | 0,4 | 0,4 | 0 | 0 | 0,4 | 0,4 |
| | Wasser | 39,6 | 39,6 | 39,6 | 39,6 | 39,6 | 39,6 |
| | | | | | | | |
| | **Gesamt** | 100 | 100 | 100 | 100 | 100 | 100 |

Die Beispiele A1 und A3 sind Vergleichsbeispiele.

Die in der Tabelle angegebenen Werte sind Gewichtsangaben in Gramm (g).

### Herstellung der O/W-Emulsion/Gelcreme:

Die Bestandteile der Phase 1 werden zur klaren Schmelze auf 70 °C erwärmt. Nach dem Abkühlen auf 40 °C wird die Phase 1 in die auf 40 °C erwärmte Phase 2 einemulgiert. Die Mischung wird bei 3000 U/Min für 5 Minuten homogenisiert. Nach Abkühlen auf Raumtemperatur wird Phase 3 bei 1000 U/min unter die entstandene Emulsion gemischt.

### Herstellung der Schaumformulierung:

90 g der Emulsion werden in Aluminiummonoblockdosen gegeben und mit 10,00 g Treibgas (Propan-Butan-Gemisch) beaufschlagt.

### Schaumerzeugung:

Ein Cremeschaum entsteht bei der Entnahme der Schaumformulierung aus der Druckgaspackung mittels eines geeigneten Ventils mit aufgesetztem Schaumapplikator.

Folgende Schaumqualitäten wurden mit den Formulierungen der Beispiele A1 bis A6 erzielt:

| | **Beispiel A1** | **Beispiel A2** | **Beispiel A3** | **Beispiel A4** | **Beispiel A5** | **Beispiel A6** |
|---|---|---|---|---|---|---|
| **Schaumqualität** | **--¹** | **+/-³** | **-²** | **+/-³** | **+/-³** | **+/-³** |

| | | | | | | |
|---|---|---|---|---|---|---|
| ¹ "--" bedeutet: sehr grobporiger Schaum der innerhalb < 1 min zerfällt; ² "-" bedeutet: grobporiger Schaum der innerhalb < 1 min zerfällt; ³ "+/-" bedeutet: grob- bis feinporiger Schaum der innerhalb 1 - 2 min zerfällt. | | | | | | |

Bei Formulierungen, deren Schaumstabilität mit "+/-" gekennzeichnet ist, lässt sich die Schaumqualität durch einen höheren Gehalt an Emulgator-Copolymer (bei gleichzeitiger Anwesenheit einer Festsubstanz in der Ölphase) in ein "+" verwandeln.

### 6.2. Beispiele B:

| | | **Beispiel B1** | **Beispiel B2** | **Beispiel B3** | **Beispiel B4** | **Beispiel B5** | **Beispiel B6** | **Beispiel B7** |
|---|---|---|---|---|---|---|---|---|
| | | | | | | | | |
| **Phase 1:** | Cetiol V | 7,5 | 0,0 | 7,5 | 5,0 | 5,0 | 5,0 | 5,0 |
| | Eutanol G | 7,5 | 0,0 | 0,0 | 0 | 0 | 0 | 0 |
| | Stearinsäure | 3,0 | 3,0 | 3,0 | 3,0 | 3,0 | 3,0 | 3,0 |
| | Paraffin | 0,0 | 7,5 | 7,5 | 5,0 | 5,0 | 5,0 | 5,0 |
| | Miglyol 812 | 0,0 | 7,5 | 0,0 | 5,0 | 5,0 | 5,0 | 5,0 |
| | Xanthan Gum | 0,2 | 0,2 | 0,2 | 0,2 | 0,2 | 0,20 | 0,20 |
| | Cosmedia SP | 0,1 | 0,1 | 0,1 | 0,2 | 0,2 | 0,20 | 0,20 |
| | | | | | | | | |
| **Phase 2:** | Wasser (ad 100) | 65,3 | 65,3 | 65,3 | 73,7 | 73,7 | 73,7 | 73,7 |
| | Harnstoff | 11,0 | 11,0 | 11,0 | 5,0 | 5,0 | 5,0 | 5,0 |
| | Propylenglykol | 2,5 | 2,5 | 2,5 | 0 | 0 | 0 | 0 |
| | Glycerol 85% | 2,5 | 2,5 | 2,5 | 2,5 | 2,5 | 2,5 | 2,5 |
| | Aristoflex AVC | 0,4 | 0,4 | 0,4 | 0,4 | 0,0 | 0,0 | 0,0 |
| | Sepinov EMT 10 | 0,0 | 0,0 | 0,0 | 0,0 | 0,4 | 0,0 | 0,0 |
| | Seppic 8732 MP | 0,0 | 0,0 | 0,0 | 0,0 | 0,0 | 0,4 | 0,0 |
| | Seppic 8947 MP | 0,0 | 0,0 | 0,0 | 0,0 | 0,0 | 0,0 | 0,4 |
| | | | | | | | | |
| | **Gesamt** | 100 | 100 | 100 | 100 | 100 | 100 | 100 |

Die Beispiele B2 und B5 sind Vergleichsbeispiele.

Die in der Tabelle angegebenen Werte sind Gewichtsangaben in Gramm (g).

### Herstellung der O/W-Emulsion/Gelcreme:

Die Lipid-Bestandteile der Phase 1 werden zur klaren Schmelze auf 70 °C erwärmt. Nach dem Abkühlen auf 40 °C werden die beiden Polymere Xanthan Gum und Cosmedia SP im Öl dispergiert.

Zur Herstellung der Phase 2 werden Harnstoff, Propylenglykol und Glycerin zum auf 40 °C erwärmten Wasser hinzugefügt und darin gelöst bzw. damit vermischt. Zur wässrigen Lösung bzw. Mischung wird das Emulgator-Copolymer hinzugefügt ("Aristoflex AVC" in Beispielen B1-B4; "Sepinov EMT 10" in Beispiel B5; "Seppic 8732 MP" in Beispiel B6 bzw. "Seppic 8947 MP" in Beispiel B7) und unter Rühren in Lösung gebracht.

Bei 40 °C und 1000 U/min wird Phase 1 in Phase 2 einemulgiert. Danach wird die Emulsion/ Gelcreme auf Raumtemperatur abgekühlt.

### Herstellung der Schaumformulierung:

90 g der Emulsion werden in Aluminiummonoblockdosen gegeben und mit 10,00 g Treibgas (Propan-Butan-Gemisch) beaufschlagt.

### Schaumerzeugung:

Ein Cremeschaum entsteht bei der Entnahme der Schaumformulierung aus der Druckgaspackung mittels eines geeigneten Ventils mit aufgesetztem Schaumapplikator.

Folgende Schaumqualitäten wurden mit den Formulierungen der Beispiele B1 bis B7 erzielt:

| | **Beispiel B1** | **Beispiel B2** | **Beispiel B3** | **Beispiel B4** | **Beispiel B5** | **Beispiel B6** | **Beispiel B7** |
|---|---|---|---|---|---|---|---|
| **Schaumqualität** | +/-² | -¹ | +/-² | +³ | -¹ | +/-² | +/-² |

| | | | | | | | |
|---|---|---|---|---|---|---|---|
| ¹"-" bedeutet: grobporiger Schaum der innerhalb < 1 min zerfällt; ² "+/-" bedeutet: grob- bis feinporiger Schaum der innerhalb 1 - 2 min zerfällt; ³ "+" bedeutet: feinporiger Schaum der innerhalb > 2 min zerfällt. | | | | | | | |

Bei Formulierungen, deren Schaumstabilität mit "+/-" gekennzeichnet ist, lässt sich die Schaumqualität durch einen höheren Gehalt an Emulgator-Copolymer (bei gleichzeitiger Anwesenheit einer Festsubstanz in der Ölphase) in ein "+" verwandeln.

## Patentansprüche

1. Schaum, der für einen Zeitraum von mindestens 1 min nicht zerfällt, umfassend eine Emulsion vom Typ Öl in Wasser, diese umfassend eine Ölphase und eine Wasserphase, wobei die Emulsion weniger als 0,5 Gew.-% herkömmlicher Emulgatoren enthält, wobei herkömmliche Emulgatoren alle amphiphilen Substanzen mit einer Molmasse von < 5000 g/mol sind, die in höherer Konzentration Mizellen bilden können, und/oder in der Emulsion nicht als Feststoff vorliegen,
und wobei die Emulsion umfasst:
a) mindestens einen Feststoffemulgator, und
b) mindestens ein grenzflächenaktives, ionisches Polymer mit einem Molekulargewicht von größer als 5000 g/mol, wobei das ionische Polymer ein Copolymer ist, das als Monomereinheiten
- ein ionisches Monomer (M1), und
- mindestens ein weiteres Monomer
enthält.

2. Schaumformulierung, die entweder mit verflüssigtem Treibgas in einen Druckbehälter abgefüllt ist oder ohne Treibgas in einen anderen Behälter als einen Druckbehälter abgefüllt ist, der es ermöglicht, bei Abgabe der Schaumformulierung einen Schaum zu erzeugen, der für einen Zeitraum von mindestens 1 min nicht zerfällt, umfassend eine Emulsion vom Typ Öl in Wasser, diese umfassend eine Ölphase und eine Wasserphase, wobei die Emulsion weniger als 0,5 Gew.-% herkömmlicher Emulgatoren enthält, wobei herkömmliche Emulgatoren alle amphiphilen Substanzen mit einer Molmasse von < 5000 g/mol sind, die in höherer Konzentration Mizellen bilden können, und/oder in der Emulsion nicht als Feststoff vorliegen,
und wobei die Emulsion umfasst:
a) mindestens einen Feststoffemulgator, und
b) mindestens ein grenzflächenaktives, ionisches Polymer mit einem Molekulargewicht von größer als 5000 g/mol, wobei das ionische Polymer ein Copolymer ist, das als Monomereinheiten
- ein ionisches Monomer (M1), und
- mindestens ein weiteres Monomer
enthält.

3. Schaumformulierung gemäß Anspruch 2,
wobei das mindestens eine weitere Monomer eine andere Polarität besitzt als das ionische Monomer (M1).

4. Schaumformulierung gemäß Anspruch 2 oder 3,
wobei das mindestens eine weitere Monomer ausgewählt ist aus der Gruppe bestehend aus ionischen Monomeren, nicht-ionischen Monomeren, und Mischungen davon.

5. Schaumformulierung gemäß einem der Ansprüche 2 bis 4,
wobei das ionische Monomer (M1) freie, partiell neutralisierte oder vollständig neutralisierte saure funktionelle Gruppen enthält, wobei die sauren funktionellen Gruppen vorzugsweise ausgewählt sind aus der Gruppe bestehend aus Sulfonsäuregruppen, Carbonsäuregruppen, Phosphorsäuregruppen, Phosphonsäuregruppen und Mischungen davon.

6. Schaumformulierung gemäß einem der Ansprüche 2 bis 5,
wobei das ionische Monomer (M1) ausgewählt ist aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze, Ammoniumsalze oder Alkanolammoniumsalze; oder als Anhydrid vorliegen können, und Mischungen davon.

7. Schaumformulierung gemäß einem der Ansprüche 2 bis 6,
wobei das ionische Monomer (M1) eine Acrylamidoalkylsulfonsäure der allgemeinen Formel (1) ist, 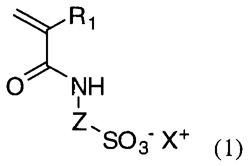 wobei R₁ ausgewählt ist aus der Gruppe bestehend aus Wasserstoff, Methyl oder Ethyl, Z ein (C₁-C₈)-Alkylen ist, das unsubstituiert oder mit einem oder mehreren (C₁-C₄)-Alkylresten substituiert sein kann, und X⁺ ausgewählt ist aus der Gruppe bestehend aus H⁺, einem Alkalimetallion, einem Erdalkalimetallion, einem Ammoniumion, einem Alkanolammoniumion, oder Mischungen davon.

8. Schaumformulierung gemäß einem der Ansprüche 2 bis 7,
wobei das ionische Monomer (M1) 2-Acrylamido-2-methyl-propansulfonsäure der allgemeinen Formel (2) ist 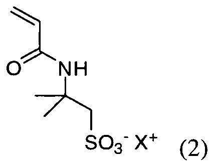 wobei X⁺ ausgewählt ist aus der Gruppe bestehend aus H⁺, einem Alkalimetallion, einem Erdalkalimetallion, einem Ammoniumion, einem Alkanolammoniumion, oder Mischungen davon.

9. Schaumformulierung gemäß einem der Ansprüche 2 bis 8,
wobei das mindestens eine weitere Monomer mindestens ein nicht-ionisches Monomer umfasst ausgewählt aus der Gruppe bestehend aus Styrolen, Chlorstyrolen, Di-(C₁-C₃₀)-Alkylaminostyrolen, Vinylchloriden, Isoprenen, Vinylalkoholen, Vinylmethylethern, (C₁-C₃₀)-Carbonsäurevinylestern, bevorzugt Vinylacetaten und Vinylpropionaten; Acrylsäureestern, Methacrylsäureestern, Maleinsäureestern, Fumarsäureestern, Crotonsäureestern; insbesondere lineare und verzweigte (C₁-C₃₀)-Alkylester der Acrylsäure, Methacrylsäure, Maleinsäure, Fumarsäure und Crotonsäure; lineare und verzweigte (C₁-C₃₀)-Hydroxyalkylester der Acrylsäure, Methacrylsäure, Maleinsäure, Fumarsäure und Crotonsäure; ethoxylierte (C₁-C₃₀)-Alkylester der Acrylsäure, Methacrylsäure, Maleinsäure, Fumarsäure und Crotonsäure mit von 1 bis 40 Ethylenoxideinheiten; Acrylamiden, insbesondere N,N-Di-(C₁-C₃₀)-Alkylacrylamiden, Methacrylamiden, insbesondere N,N-Di-(C₁-C₃₀)-Alkylmethacrylamiden, cyclischen und linearen N-Vinylcarbonsäureamiden mit einer Kohlenstoffkette von 2 bis 9 Kohlenstoffatomen, bevorzugt N-Vinylpyrrolidon; und Mischungen davon.

10. Schaumformulierung gemäß einem der Ansprüche 2 bis 9,
wobei das mindestens eine weitere Monomer mindestens ein ionisches Monomer umfasst ausgewählt aus der Gruppe bestehend aus Acrylsäuren, Methacrylsäuren, Crotonsäuren, Maleinsäuren, Fumarsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, partiell oder vollständig neutralisiert in Form ihrer Salze, bevorzugt der Alkalimetallsalze, Erdalkalimetallsalze oder Ammoniumsalze; oder als Anhydrid vorliegen können, und Mischungen davon.

11. Schaumformulierung gemäß einem der Ansprüche 2 bis 10,
wobei das mindestens eine grenzflächenaktive, ionische Polymer ausgewählt ist aus der Gruppe bestehend aus Acryloyldimethyltaurat/ Vinylpyrrolidon-Copolymer, Natriumacrylat/ Acryloyldimethyltaurad Dimethylacrylamid-Crosspolymer, Hydroxyethylacrylat/ Natriumacryloyldimethyltaurat-Copolymer, Natriumacrylat/ Natriumacryloyldimethyltaurat-Copolymer, und Mischungen davon.

12. Verwendung einer Emulsion vom Typ Öl in Wasser, umfassend eine Ölphase und eine Wasserphase, wobei die Emulsion weniger als 0,5 Gew.-% herkömmlicher Emulgatoren enthält, wobei herkömmliche Emulgatoren alle amphiphilen Substanzen mit einer Molmasse von < 5000 g/mol sind, die in höherer Konzentration Mizellen bilden können, und/oder in der Emulsion nicht als Feststoff vorliegen,
die Emulsion umfassend:
a) mindestens einen Feststoffemulgator, und
b) mindestens ein grenzflächenaktives, ionisches Polymer mit einem Molekulargewicht von größer als 5000 g/mol, wobei das ionische Polymer ein Copolymer ist, das als Monomereinheiten
- ein ionisches Monomer (M1), und
- mindestens ein weiteres Monomer
enthält,
für die Herstellung einer Schaumformulierung gemäß einem der Ansprüche 2 bis 11.

13. Verwendung mindestens eines grenzflächenaktiven, ionischen Polymers mit einem Molekulargewicht von größer als 5000 g/mol, wobei das ionische Polymer ein Copolymer ist, das als Monomereinheiten
- ein ionisches Monomer (M1), und
- mindestens ein weiteres Monomer
enthält,
in Kombination mit mindestens einem Feststoffemulgator zur Stabilisierung von Schaumformulierungen gemäß einem der Ansprüche 2 bis 11.

14. Verwendung einer Schaumformulierung gemäß einem der Ansprüche 2 bis 11 als Träger für einen Wirkstoff, als Hautpflegemittel, als Hautreinigungsmittel oder zur Herstellung eines Sonnenschutzmittels, Kosmetikums, Medizinprodukts oder Arzneimittels.

15. Verfahren zur Herstellung einer Schaumformulierung gemäß einem der Ansprüche 2 bis 11, umfassend die Schritte:
a) Herstellen einer Emulsion vom Typ Öl in Wasser
b) Abfüllen der Emulsion mit Treibgas in einen Druckbehälter, oder
c) Abfüllen der Emulsion in einen anderen als einen Druckbehälter, der bei Abgabe der Emulsion einen Schaum erzeugt.

## Claims

1. Foam, which does not collapse for a time period of at least 1 min, comprising an emulsion of the oil in water type, said emulsion comprising an oil phase and a water phase, wherein the emulsion contains less than 0.5 wt.-% of conventional emulsifiers, wherein conventional emulsifiers are all amphiphilic substances with a molecular weight of < 5000 g/mol, which in higher concentrations can form micelles, and/or are not present in the emulsion as solid,
and wherein the emulsion comprises:
a) at least one solid emulsifier, and
b) at least one surface-active, ionic polymer with a molecular weight of more than 5000 g/mol, wherein the ionic polymer is a co-polymer, which contains as monomer units
- an ionic monomer (M1), and
- at least one further monomer.

2. Foam formulation which is either filled into a pressurized container with a liquefied propellant or without propellant into a container other than a pressurized container that allows for the formation of a foam upon dispensing of the emulsion which does not collapse for a time period of at least 1 min, comprising an emulsion of the oil in water type, said emulsion comprising an oil phase and a water phase, wherein the emulsion contains less than 0.5 wt.-% of conventional emulsifiers, wherein conventional emulsifiers are all amphiphilic substances with a molecular weight of < 5000 g/mol, which in higher concentrations can form micelles, and/or are not present in the emulsion as solid,
and wherein the emulsion comprises:
a) at least one solid emulsifier, and
b) at least one surface-active, ionic polymer with a molecular weight of more than 5000 g/mol, wherein the ionic polymer is a co-polymer, which contains as monomer units
- an ionic monomer (M1), and
- at least one further monomer.

3. Foam formulation according to claim 2,
wherein the at least one further monomer has a different polarity than the ionic monomer (M1).

4. Foam formulation according to claim 2 or 3,
wherein the at least one further monomer is selected from the group consisting of ionic monomers, non-ionic monomers, and mixtures thereof.

5. Foam formulation according to any one of claims 2 to 4,
wherein the ionic monomer (M1) contains free, partially neutralized or completely neutralized acid functional groups, wherein the acid functional groups are preferably selected from the group consisting of sulfonic acid groups, carboxylic acid groups, phosphoric acid groups, phosphonic acid groups and mixtures thereof.

6. Foam formulation according to any one of claims 2 to 5,
wherein the ionic monomer (M1) is selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts, preferably the alkali metal salts, alkaline-earth metal salts, ammonium salts or alkanol ammonium salts; or as anhydride, and mixtures thereof.

7. Foam formulation according to any one of claims 2 to 6,
wherein the ionic monomer (M1) is an acrylamido alkylsulfonic acid having the general formula (1), 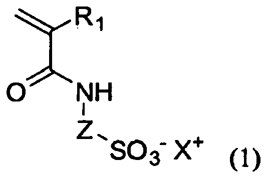 wherein R₁ is selected from the group consisting of hydrogen, methyl or ethyl, Z is a (C₁-C₈)-alkylene, which may be unsubstituted or substituted with one or more (C₁-C₄)-alkyl groups, and X⁺ is selected from the group consisting of H⁺, an alkali metal ion, an alkaline-earth metal ion, an ammonium ion, an alkanol ammonium ion, or mixtures thereof.

8. Foam formulation according to any one of claims 2 to 7,
wherein the ionic monomer (M1) is 2-acrylamido-2-methylpropane sulfonic acid having the general formula (2) 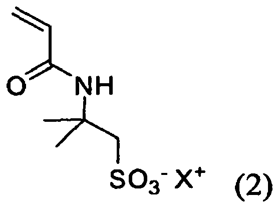 wherein X⁺ is selected from the group consisting of H⁺, an alkali metal ion, an alkaline-earth metal ion, an ammonium ion, an alkanol ammonium ion, or mixtures thereof.

9. Foam formulation according to any one of claims 2 to 8,
wherein the at least one further monomer comprises at least one non-ionic monomer selected from the group consisting of styrenes, chlorostyrenes, di-(C₁-C₃₀)-alkylamino styrenes, vinyl chlorides, isoprenes, vinyl alcohols, vinyl methyl ethers, (C₁-C₃₀)-carboxylic acid vinyl esters, preferably vinyl acetates and vinyl propionates; acrylic acid esters, methacrylic acid esters, maleic acid esters, fumaric acid esters, crotonic acid esters; in particular linear and branched (C₁-C₃₀)-alkyl esters of acrylic acid, methacrylic acid, maleic acid, fumaric acid and crotonic acid; linear and branched (C₁-C₃₀)-hydroxyalkyl esters of acrylic acid, methacrylic acid, maleic acid, fumaric acid and crotonic acid; ethoxylated (C₁-C₃₀)-alkyl esters of acrylic acid, methacrylic acid, maleic acid, fumaric acid and crotonic acid with from 1 to 40 ethylene oxide units; acrylamides, in particular N,N-di(C₁-C₃₀)-alkyl acrylamides, methacrylamides, in particular N,N-di-(C₁-C₃₀)-alkyl methacrylamides, cyclic and linear N-vinyl carboxylic acid amides with a carbon chain having from 2 to 9 carbon atoms, preferably N-vinylpyrrolidone; and mixtures thereof.

10. Foam formulation according to any one of claims 2 to 9,
wherein the at least one further monomer comprises at least one ionic monomer selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts, preferably the alkali metal salts, alkaline-earth metal salts or ammonium salts; or as anhydride, and mixtures thereof.

11. Foam formulation according to any one of claims 2 to 10,
wherein the at least one surface active, ionic polymer is selected from the group consisting of acryloyldimethyltaurate/vinylpyrrolidone copolymer, sodium acrylate/acryloyldimethyltaurate/dimethylacrylamide crosspolymer, hydroxyethyl acrylate/sodium acryloyldimethyltaurate copolymer, sodium acrylate/sodium acryloyldimethyltaurate copolymer, and mixtures thereof.

12. Use of an emulsion of the oil in water type, comprising an oil phase and a water phase, wherein the emulsion contains less than 0.5 wt.-% of conventional emulsifiers, wherein conventional emulsifiers are all amphiphilic substances with a molecular weight of < 5000 g/mol, which in higher concentration can form micelles, and/or are not present in the emulsion as solid,
the emulsion comprises:
a) at least one solid emulsifier, and
b) at least one surface-active, ionic polymer with a molecular weight of more than 5000 g/mol, wherein the ionic polymer is a co-polymer comprising as monomer units
- an ionic monomer (M1), and
- at least one further monomer,
for the manufacture of a foam formulation according to any one of claims 2 to 11.

13. Use of at least one surface active, ionic polymer with a molecular weight of more than 5000 g/mol, wherein the ionic polymer is a co-polymer comprising as monomer units
- an ionic monomer (M1), and
- at least one further monomer,
in combination with at least one solid emulsifier for stabilizing foam formulations according to any one of claims 2 to 11.

14. Use of a foam formulation according to any one of claims 2 to 11 as carrier for an active agent, as skincare agent, as skin cleaning agent or for the manufacture of a sunscreen, a cosmetic, a medical product or a medicament.

15. Method of manufacture of a foam formulation according to any one of claims 2 to 11, comprising the steps:
a) preparing an emulsion of the oil in water type
b) filling the emulsion with propellant into a pressurized container, or
c) filling the emulsion into a container other than a pressurized container that produces a foam upon dispensing of the emulsion.

## Revendications

1. Mousse qui ne se désintègre pas pendant un laps de temps d'au moins 1 minute, comprenant une émulsion huile dans l'eau, celle-ci comprenant une phase huileuse et une phase aqueuse, dans laquelle l'émulsion contient moins de 0,5 % en poids d'émulsifiants courants ; les émulsifiants courants étant toutes les substances amphiphiles d'une masse molaire < 5 000 g/mol qui, en concentration élevée, peuvent former des micelles et/ou ne sont pas présentes comme composant solide dans l'émulsion,
et l'émulsion comprenant :
a) au moins un émulsifiant solide et
b) au moins un polymère tensioactif ionique d'un poids moléculaire supérieur à 5 000 g/mol, le polymère ionique étant un copolymère qui contient comme unités monomères
- un monomère ionique (M1) et
- au moins un autre monomère.

2. Formulation d'une mousse qui est conditionnée soit avec du gaz propulseur liquéfié dans un réservoir sous pression, soit sans gaz propulseur dans un autre réservoir qu'un réservoir sous pression permettant, lors de la libération de la formulation de mousse, de produire une mousse qui ne se désintègre pas pendant un laps de temps d'au moins 1 minute, comprenant une émulsion huile dans l'eau, celle-ci comprenant une phase huileuse et une phase aqueuse, dans laquelle l'émulsion contient moins de 0,5 % en poids d'émulsifiants courants ; les émulsifiants courants étant toutes les substances amphiphiles d'une masse
molaire < 5 000 g/mol qui, en concentration élevée, peuvent former des micelles et/ou ne sont pas présentes comme composant solide dans l'émulsion, et l'émulsion comprenant :
a) au moins un émulsifiant solide et
b) au moins un polymère tensioactif ionique d'un poids moléculaire supérieur à 5 000 g/mol, le polymère ionique étant un copolymère qui contient comme unités monomères
- un monomère ionique (M1) et
- au moins un autre monomère.

3. Formulation d'une mousse selon la revendication 2,
dans laquelle le au moins un autre monomère possède une autre polarité que le monomère ionique (M1).

4. Formulation d'une mousse selon la revendication 2 ou 3,
dans laquelle le au moins un autre monomère est choisi parmi le groupe constitué de monomères ioniques, de monomères non ioniques et de mélanges de ceux-ci.

5. Formulation d'une mousse selon l'une des revendications 2 à 4,
dans laquelle le monomère ionique (M1) contient des groupements fonctionnels acides libres, partiellement ou totalement neutralisés, les groupements fonctionnels acides étant de préférence choisis parmi le groupe constitué de groupements acide sulfonique, de groupements acide carboxylique, de groupements acide phosphorique, de groupements acide phosphonique, et de mélanges de ceux-ci.

6. Formulation d'une mousse selon l'une des revendications 2 à 5,
dans laquelle le monomère ionique (M1) est choisi parmi le groupe constitué d'acides acryliques, d'acides méthacryliques, d'acides crotoniques, d'acides maléiques, d'acides fumariques, d'acides styrène-sulfoniques, d'acides vinylsulfoniques, d'acides vinylphosphoniques, d'acides allyl-sulfuriques, d'acides méthallyliques sulfoniques, d'acides acrylamido alkylsulfoniques, qui peuvent être présents respectivement comme acide libre, partiellement ou totalement neutralisés sous la forme de leurs sels, de préférence des sels de métal alcalin, sels de métal alcalino-terreux, sels d'ammonium ou sels d'alcanol ammonium ; ou bien sous la forme d'un anhydride, et de mélanges de ceux-ci.

7. Formulation d'une mousse selon l'une des revendications 2 à 6,
dans laquelle le monomère ionique (M1) est un acide acrylamido alkylsulfonique de la formule générale (1), 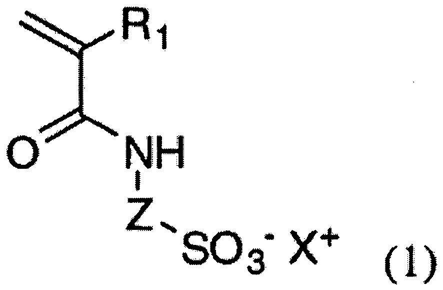 dans laquelle R₁ est choisi parmi le groupe constitué d'hydrogène, de méthyle ou d'éthyle, Z est un alkylène (C₁-C₈) qui peut être non substitué ou substitué par un ou plusieurs radicaux alkyles (C₁-C₄), et X⁺ est choisi parmi le groupe constitué de H⁺, d'un ion de métal alcalin, d'un ion de métal alcalino-terreux, d'un ion d'ammonium, d'un ion d'alcanol ammonium, ou de mélanges de ceux-ci.

8. Formulation d'une mousse selon l'une des revendications 2 à 7,
dans laquelle le monomère ionique (M1) est un acide 2-acrylamido-2-méthylepropanesulfonique de la formule générale (2) 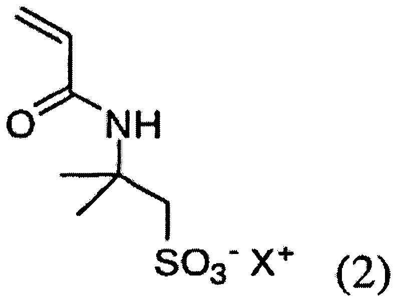 dans laquelle X⁺ est choisi parmi le groupe constitué de H⁺, d'un ion de métal alcalin, d'un ion de métal alcalino-terreux, d'un ion d'ammonium, d'un ion d'alcanol ammonium, ou de mélanges de ceux-ci.

9. Formulation d'une mousse selon l'une des revendications 2 à 8,
dans laquelle le au moins un autre monomère comprend au moins un monomère non ionique, choisi parmi le groupe constitué de styrènes, de chlorostyrènes, de di(C₁-C₃₀)-alkylaminostyrènes, de chlorures vinyliques, d'isoprènes, d'alcools vinyliques, de méthyléthers vinyliques, d'esters vinyliques d'acide carboxylique (C₁-C₃₀), de préférence acétates de vinyle et propionates de vinyle ; d'esters d'acide acrylique, d'esters d'acide méthacrylique, d'esters d'acide maléique, d'esters d'acide fumarique et d'esters d'acide crotonique ; en particulier des esters d'alkyle linéaires et dérivés (C₁-C₃₀) de l'acide acrylique, l'acide méthacrylique, l'acide maléique, l'acide fumarique et l'acide crotonique ; d'esters d'hydroxyalkyle linéaires et dérivés (C₁-C₃₀) de l'acide acrylique, l'acide méthacrylique, l'acide maléique, l'acide fumarique et l'acide crotonique ; d'esters d'alkyle éthoxylés (C₁-C₃₀) de l'acide acrylique, l'acide méthacrylique, l'acide maléique, l'acide fumarique et l'acide crotonique avec de 1 à 40 unités d'oxyde d'éthylène ; amides acryliques, en particulier diamides N,N- (C₁-C₃₀) alkylacryliques, amides méthacryliques, en particulier diamides alkylméthacryliques N,N (C₁-C₃₀), amides cycliques et linéaires d'acide carboxylique de vinyle N avec une chaîne carbonée constituée de 2 à 9 atomes de carbone, de préférence N-vinylpyrrolidone ; et de mélanges de ceux-ci.

10. Formulation d'une mousse selon l'une des revendications 2 à 9,
dans laquelle le au moins un autre monomère comprend au moins un monomère ionique, choisi parmi le groupe constitué d'acides acryliques, d'acides méthacryliques, d'acides crotoniques, d'acides maléiques, d'acides fumariques, d'acides styrène-sulfoniques, d'acides vinylsulfoniques, d'acides vinylphosphoniques, d'acides allyl-sulfoniques, d'acides méthallyliques sulfoniques, d'acides acrylamido alkylsulfoniques qui peuvent être présents respectivement comme un acide libre, partiellement ou totalement neutralisés sous la forme de leurs sels, de préférence des sels de métal alcalin, sels de métal alcalino-terreux ou sels d'ammonium ; ou bien comme un anhydride, et de mélanges de ceux-ci.

11. Formulation d'une mousse selon l'une des revendications 2 à 10,
dans laquelle le au moins un polymère tensioactif ionique est choisi parmi le groupe constitué d'un copolymère de vinylpyrrolidone/ taurate de diméthyle d'acryloyle, d'un polymère réticulé d'acrylate de sodium/ taurate de diméthyle d'acryloyle/ diméthylacrylamide, d'un copolymère d'acrylate d'hydroxyéthyle/ taurate de diméthyle d'acryloyle de sodium, d'un copolymère d'acrylate de sodium/ taurate de diméthyle d'acryloyle de sodium, et de mélanges de ceux-ci.

12. Utilisation d'une émulsion de type huile dans l'eau, comprenant une phase huileuse et une phase aqueuse, dans laquelle l'émulsion contient moins de 0,5 % en poids d'émulsifiants courants ; les émulsifiants courants étant toutes les substances amphiphiles d'une masse molaire < 5 000 g/mol qui, en concentration élevée, peuvent former des micelles et/ou ne sont pas présentes comme composant solide dans l'émulsion,
l'émulsion comprenant :
a) au moins un émulsifiant solide et
b) au moins un polymère tensioactif ionique d'un poids moléculaire supérieur à 5 000 g/mol, le polymère ionique étant un copolymère qui contient comme unités monomères
- un monomère ionique (M1) et
- au moins un autre monomère,
pour la fabrication d'une formulation de mousse selon l'une des revendications 2 à 11.

13. Utilisation d'au moins un polymère tensioactif ionique d'un poids moléculaire supérieur à 5 000 g/mol, le polymère ionique étant un copolymère qui contient comme unités monomères
- un monomère ionique (M1) et
- au moins un autre monomère,
en combinaison avec au moins un émulsifiant solide pour la stabilisation de formulations de mousse selon l'une des revendications 2 à 11.

14. Utilisation d'une formulation de mousse selon l'une des revendications 2 à 11 comme support de principe actif, comme produit de soin pour la peau, comme produit nettoyant pour la peau ou pour la fabrication d'un produit de protection solaire, d'un produit cosmétique, d'un produit médical ou d'un médicament.

15. Procédé de fabrication d'une formulation de mousse selon l'une des revendications 2 à 11, comprenant les étapes suivantes :
a) Fabrication d'une émulsion huile dans l'eau
b) Conditionnement de l'émulsion avec un gaz propulseur dans un réservoir sous pression ou
c) Conditionnement de l'émulsion dans un autre réservoir qu'un réservoir sous pression qui génère une mousse lors de la libération de l'émulsion.
